# EUROPÄISCHE PATENTSCHRIFT

(11) **EP 3 642 253 B1**
(45) Veröffentlichungstag und Bekanntmachung des Hinweises auf die Patenterteilung: **10.05.2023**
(21) Anmeldenummer: 18732067.6
(22) Anmeldetag: 19.06.2018
(51) Int. Cl.: C08G 18/48, C08G 18/75, C08G 18/76, C08G 18/12, C08G 18/32

(54) **BLOCKIERUNGSMITTEL FÜR AMINE, LATENTE HÄRTER UND POLYURETHANZUSAMMENSETZUNGEN**
BLOCKING AGENTS FOR AMINES, LATENT CURING AGENTS AND POLYURETHANE COMPOSITIONS
MOYEN DE BLOCAGE POUR AMINE, DURCISSEUR LATENT ET COMPOSITIONS DE POLYURÉTHANE

(30) Priorität: 19.06.2017 EP 17176691
(43) Veröffentlichungstag der Anmeldung: 29.04.2020
(73) Patentinhaber: Sika Technology AG, 6340 Baar (CH)
(72) Erfinder: BURCKHARDT, Urs, 8049 Zürich (CH); KRAMER, Andreas, 8008 Zürich (CH)
(74) Vertreter: Sika Patent Attorneys
(86) Internationale Anmeldenummer: PCT/EP2018/066179
(87) Internationale Veröffentlichungsnummer: WO 2018/234268

(56) Entgegenhaltungen:
- WO-A1-2004/013200
- US-A1- 2015 111 991

## Beschreibung

### Technisches Gebiet

Die Erfindung betrifft ein Blockierungsmittel für Amine, latente Härter und Polyurethanzusammensetzungen davon, insbesondere für die Anwendung als elastische Klebstoffe, Dichtstoffe und Beschichtungen.

### Stand der Technik

Polyurethan-Zusammensetzungen, welche durch Reaktion von Isocyanatgruppen mit Feuchtigkeit bzw. Wasser vernetzen und dabei zu Elastomeren aushärten, werden insbesondere als Klebstoffe, Dichtstoffe oder Beschichtungen in der Bau- und Fertigungsindustrie eingesetzt, beispielsweise zur Bauteilverklebung in der Montage, zur Verfüllung von Fugen, als Bodenbeschichtung oder als Dachabdichtung. Aufgrund ihrer guten Haftung und Elastizität können sie auf die Substrate einwirkende Kräfte, ausgelöst etwa durch Vibrationen oder Temperaturschwankungen, schonend dämpfen. Wenn solche Zusammensetzungen bei hoher Luftfeuchtigkeit und/oder erhöhter Temperatur eingesetzt werden, verläuft ihre Aushärtung aber oft unter störender, Haftung und Festigkeit beeinträchtigender Blasenbildung durch freigesetztes Kohlendioxid-Gas, das nicht schnell genug gelöst oder abgeleitet wird. Um die Blasenbildung zu vermindern, können den Zusammensetzungen chemisch blockierte Amine, sogenannte latente Härter, zugegeben werden, welche durch Hydrolyse Feuchtigkeit binden und dabei Aminogruppen freisetzen, welche ohne Kohlendioxid-Bildung mit den Isocyanatgruppen reagieren. Als latente Härter werden meist Verbindungen mit Aldimin-, Ketimin- oder Oxazolidin-Gruppen verwendet. Die bekannten latenten Härter bringen aber oft den Nachteil mit sich, bei Raumtemperatur hochviskose Öle oder Feststoffe zu sein und somit erhöhte Anforderungen an ihre Handhabung zu stellen, indem sie zum Beispiel aufgeschmolzen oder gelöst werden müssen. Weiterhin können sie eine ungenügende Latenz aufweisen, sodass sie bei der Lagerung in Isocyanatgruppen-haltigen Zusammensetzungen vorzeitige Vernetzungsreaktionen der Isocyanat-gruppen auslösen und damit die Lagerstabilität der Zusammensetzung herabsetzen und/oder deren Aushärtung so stark beschleunigen, dass sich eine zu kurze Offenzeit und damit ein zu kleines Verarbeitungsfenster ergibt. Zudem führen viele der bekannten latenten Härter bei der Aushärtung zu störenden Immissionen, verursacht durch leichtflüchtige, geruchsintensive Aldehyde oder Ketone, welche im latenten Härter als Blockierungsmittel dienen und durch Hydrolyse freigesetzt werden.

Aus WO 2004/013200 sind Polyurethan-Zusammensetzungen mit latenten Härtern bekannt, deren Blockierungsmittel nicht flüchtig sind und somit in der ausgehärteten Zusammensetzung verbleiben. Diese Zusammensetzungen sind vollkommen geruchlos und können deshalb auch in Innenräumen oder bei grossflächigen Anwendungen eingesetzt werden, ohne Immissionsprobleme zu verursachen. Sie weisen aber verschiedene Nachteile auf. Das nichtflüchtige Blockierungsmittel wirkt in der ausgehärteten Zusammensetzung als Weichmacher, was die mechanische Festigkeit bzw. Steifigkeit herabsetzen kann. Der erhöhte Weichmachergehalt führt oft zu verstärkter Weichmachermigration, was sich in Ausschwitzen (Bleeding), klebriger, leicht anschmutzender Oberfläche der Zusammensetzung, Verunreinigung oder Schädigung der Substrate oder in Problemen beim Überdecken, insbesondere Überstreichen, Überlackieren, Überbeschichten oder Überkleben, der Zusammensetzung zeigen kann. Ein nichtflüchtiges anstelle eines flüchtigen Blockierungsmittel führt schliesslich auch zu deutlich höheren Kosten. Einerseits wird aufgrund seiner höheren Masse mengenmässig mehr Blockierungsmittel benötigt, und andererseits ist seine Aufreinigung aufwendiger, da es beispielsweise im Hochvakuum bei hohen Temperaturen destilliert werden muss, was aufwendige Apparaturen erfordert, lange dauert und wegen Polymerbildung und anderen Zersetzungsreaktionen bei den erforderlichen hohen Temperaturen oft geringe Ausbeuten liefert.

WO 2013/179915 beschreibt verzweigte langkettige Alkylbenzaldehyde, welche in destillierter bzw. fraktionierter Form als Blockierungsmittel in Amin-Komponenten von Epoxidharz-Zusammensetzungen eingesetzt werden. Bei der Destillation bzw. Fraktionierung wie in Example 2 beschrieben werden die Aldehyde über Kopf destilliert und als Hauptfraktion mit einem Siedepunkt von 187 bis 230°C bei 8 Torr aufgefangen und weiterverwendet. Dabei wurden leichtflüchtige Anteile und insbesondere auch die höhersiedenden Nebenprodukte abgetrennt.

### Darstellung der Erfindung

Aufgabe der vorliegenden Erfindung ist es daher, ein kostengünstiges Blockierungsmittel für Amine zur Verfügung zu stellen, welches die Herstellung preiswerter blockierter Amine bzw. latenter Härter ermöglicht, die bei Raumtemperatur flüssig und niedrigviskos sind, keine Immissionen und Probleme mit Weichmacher-Migration verursachen und zusammen mit Isocyanatgruppen lagerstabil sind.

Diese Aufgabe wird mit dem im Anspruch 1 beschriebenen Blockierungsmittel in Form eines Aldehydgemisches, welches als Hauptbestandteil Alkylbenzaldehyde und als Nebenbestandteil weitere, höhersiedende Alkylbenzol-Verbindungen enthält, gelöst. Das Aldehydgemisch ist insbesondere erhältlich als nicht oder nur geringfügig aufgereinigtes Rohprodukt einer Formylierung von Alkylbenzolen und ist äusserst kostengünstig; es ergibt dadurch auch deutlich preiswertere blockierte Amine, als wenn entsprechende aufgereinigte Blockierungsmittel eingesetzt werden, bei welchen die höhersiedenden Anteil entfernt wurden. Überraschenderweise werden die Eigenschaften der blockierten Amine selbst und der diese als latente Härter enthaltenden Isocyanatgruppen-haltigen Zusammensetzungen durch den Nebenbestandteil des erfindungsgemässen Blockierungsmittels nicht beeinträchtigt, sondern überraschenderweise sogar verbessert. Die erfindungsgemässen blockierten Amine sind bei Raumtemperatur typischerweise flüssig und niedrigviskos und können somit einfach und lösemittelfrei eingesetzt werden. Überraschenderweise sind sie sogar niedrigviskoser als die mit entsprechenden, nach Entfernung des höhersiedenden Nebenbestandteils aufgereinigten Blockierungsmitteln hergestellten latenten Härter, was angesichts des erhöhten Molekulargewichts und der partiellen Aldehyd-Funktionalität des Nebenbestandteils, die zu hochmolekularen Kondensationsprodukten führt, erstaunlich ist. Weiterhin weisen sie eine tiefere Kristallisationstemperatur auf und bleiben dadurch bei Lagerung in der Kälte sehr lange flüssig. Schliesslich sind sie trotz fehlender Aufreinigung des Blockierungsmittels überraschend hellfarbig und haben eine erstaunlich gute Latenz gegenüber Isocyanaten.

Mit den erfindungsgemässen latenten Härtern formulierte feuchtigkeitshärtende Polyurethanzusammensetzungen sind niedrigviskos, lange lagerfähig, gut verarbeitbar, ohne Geruchsproblematik auch in Innenräumen, für grossflächige und für emissionsarme Anwendungen einsetzbar und verfügen über eine lange Offenzeit bei schneller Aushärtung. Im ausgehärteten Zustand haben sie gute mechanische Eigenschaften mit hoher Elastizität und geringer Neigung zu Weichmachermigration. Überraschenderweise zeigen sie gegenüber analogen Zusammensetzungen mit latenten Härtern aus entsprechenden aufgereinigten Blockierungsmitteln stellenweise sogar bessere Eigenschaften in Bezug auf die Viskosität, Lagerstabilität und die mechanischen Kennwerte, insbesondere die Festigkeit.

Weitere Aspekte der Erfindung sind Gegenstand weiterer unabhängiger Ansprüche. Besonders bevorzugte Ausführungsformen der Erfindung sind Gegenstand der abhängigen Ansprüche.

### Wege zur Ausführung der Erfindung

Gegenstand der Erfindung ist die Verwendung eines Aldehydgemisches enthaltend
- 70 bis 92 Gewichts-% Aldehyde der Formel (I), wobei R für einen Alkyl-Rest mit 6 bis 20 C-Atomen steht, und 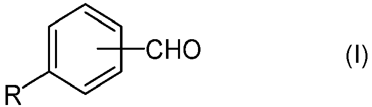
- 8 bis 30 Gewichts-% nicht der Formel (I) entsprechende Alkylbenzol-Verbindungen
als Blockierungsmittel für Amine,
dadurch gekennzeichnet, dass die nicht der Formel (I) entsprechenden Alkylbenzol-Verbindungen einen höheren Siedepunkt als die Aldehyde der Formel (I) aufweisen.

Das Aldehydgemisch enthält 70 bis 92 Gewichts-% Aldehyde der Formel (I) und 8 bis 30 Gewichts-% nicht der Formel (I) entsprechende Alkylbenzol-Verbindungen.

Die nicht der Formel (I) entsprechende Alkylbenzol-Verbindungen weisen einen höheren Siedepunkt als die Aldehyde der Formel (I) auf.

Bevorzugt steht R für Alkyl-Reste mit 8 bis 16 C-Atomen, insbesondere mit 10 bis 14 C-Atomen.

Besonders bevorzugt steht R für Alkyl-Reste mit 10 bis 14 C-Atomen, und die nicht der Formel (I) entsprechenden Alkylbenzol-Verbindungen weisen ein Molekulargewicht von mindestens 350 g/mol auf.

Bevorzugt sind die Alkyl-Reste R mehrheitlich verzweigt.

Insbesondere steht R für mehrheitlich verzweigte Alkyl-Reste mit 10 bis 14 C-Atomen. Solche Aldehyde ergeben besonders niedrigviskose blockierte Amine. Besonders bevorzugt steht R für Reste der Formel 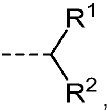 wobei R¹ und R² jeweils für Alkyl-Reste stehen und zusammen jeweils 9 bis 13 C-Atome aufweisen.

Die Formyl-Gruppe steht bevorzugt in meta- oder para-Stellung zum Rest R. Besonders bevorzugt steht die Formyl-Gruppe in para-Stellung.

Ganz besonders bevorzugt stellen die Aldehyde der Formel (I) also Aldehyde der Formel (I a) dar, wobei R¹ und R² die bereits genannten Bedeutungen aufweisen.

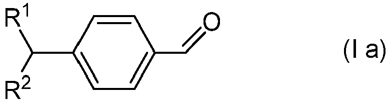

Besonders bevorzugte Aldehyde der Formel (I) sind 4-Decylbenzaldehyd, 4-Undecylbenzaldehyd, 4-Dodecylbenzaldehyd, 4-Tridecylbenzaldehyd, 4-Tetradecylbenzaldehyd, in denen die 4-Alkyl-Reste verzweigt vorliegen.

Bevorzugt sind die Aldehyde der Formel (I) ausgewählt aus 4-Decylbenzaldehyden, 4-Undecylbenzaldehyden, 4-Dodecylbenzaldehyden, 4-Tridecylbenzaldehyden und 4-Tetradecylbenzaldehyden, deren Alkyl-Reste mehrheitlich verzweigt sind.

Die nicht der Formel (I) entsprechenden Alkylbenzol-Verbindungen sind vorwiegend höhermolekular als die Aldehyde der Formel (I) und partiell Aldehydfunktionell.

Bevorzugt umfassen sie eine oder mehrere Verbindungen der Formeln 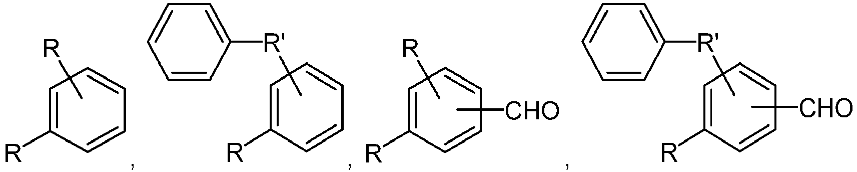 oder 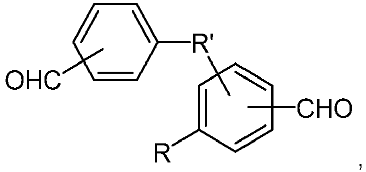 wobei R' für einen Alkylen-Rest mit 6 bis 20 C-Atomen steht und R die bereits beschriebenen Bedeutungen aufweist.

Solche höhermolekulare Verbindungen werden beim Aufreinigen eines Gemischs von Aldehyden der Formel (I) durch über Kopf Destillation bzw. Fraktionierung abgetrennt, da sie einen wesentlich höheren Siedepunkt als die entsprechenden Aldehyde der Formel (I) aufweisen, wobei sie typischerweise als sogenannter Sumpf entsorgt werden. Bei der erfindungsgemässen Verwendung werden sie aber zusammen mit den Aldehyden der Formel (I) mitverwendet, was überraschenderweise zu den beschriebenen unerwarteten Vorteilen führt.

Besonders bevorzugt enthält das Aldehydgemisch 5 bis 30 Gewichts-%, insbesondere 8 bis 30 Gewichts-%, Alkylbenzol-Verbindungen ausgewählt aus Verbindungen der Formeln 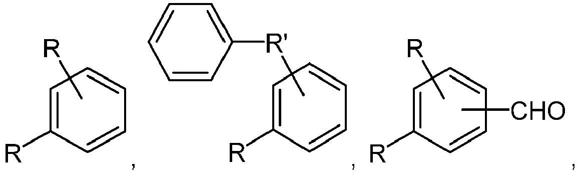 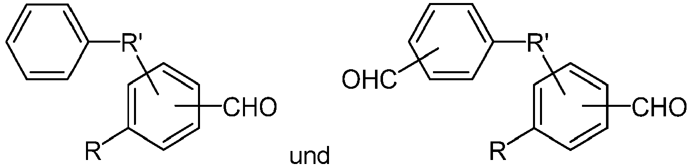 , wobei R und R' die bereits genannten Bedeutungen aufweisen.

Als "primäre Aminogruppe" wird eine Aminogruppe bezeichnet, die an einen einzigen organischen Rest gebunden ist und zwei Wasserstoffatome trägt; als "sekundäre Aminogruppe" wird eine Aminogruppe bezeichnet, die an zwei organische Reste, welche auch gemeinsam Teil eines Rings sein können, gebunden ist und ein Wasserstoffatom trägt; und als "tertiäre Aminogruppe" wird eine Aminogruppe bezeichnet, die an drei organische Reste, welche auch zu zweit oder zu dritt Teil eines oder mehrerer Ringe sein können, gebunden ist und kein Wasserstoffatom trägt.

Als "primäres Diamin" wird eine Verbindung mit zwei primären Aminogruppen bezeichnet.

Als "aromatisch" wird ein Amin oder ein Isocyanat bezeichnet, dessen Amino- oder Isocyanat-Gruppen direkt an ein aromatisches C-Atom gebunden sind. Als "aliphatisch" wird ein Amin oder ein Isocyanat bezeichnet, dessen Amino- oder Isocyanat-Gruppen direkt an ein aliphatisches C-Atom gebunden sind. Als "Silangruppe" wird eine an einen organischen Rest gebundene Silylgruppe mit ein bis drei, insbesondere zwei oder drei, hydrolysierbaren Alkoxy-Resten am Silicium-Atom bezeichnet.

Als "Molekulargewicht" wird die molare Masse (in Gramm pro Mol) eines Moleküls oder eines Molekül-Rests bezeichnet. Als "mittleres Molekulargewicht" wird das Zahlenmittel des Molekulargewichts (Mₙ) einer polydispersen Mischung von oligomeren oder polymeren Molekülen oder Molekül-Resten bezeichnet. Es wird üblicherweise mittels Gelpermeationschromatographie (GPC) gegen Polystyrol als Standard bestimmt.

Eine gestrichelte Linie in den Formeln stellt jeweils die Bindung zwischen einem Substituenten und dem zugehörigen Molekülrest dar.

Mit "Poly" beginnende Substanznamen wie Polyamin, Polyol oder Polyisocyanat bezeichnen Substanzen, die formal zwei oder mehr der in ihrem Namen vorkommenden funktionellen Gruppen pro Molekül enthalten.

Als "lagerstabil" oder "lagerfähig" wird eine Substanz oder eine Zusammensetzung bezeichnet, wenn sie bei Raumtemperatur in einem geeigneten Gebinde während längerer Zeit, typischerweise während mindestens 3 Monaten bis zu 6 Monaten und mehr, aufbewahrt werden kann, ohne dass sie sich in ihren Anwendungs- oder Gebrauchseigenschaften durch die Lagerung in einem für ihren Gebrauch relevanten Ausmass verändert.

Als "Raumtemperatur" wird eine Temperatur von 23°C bezeichnet.

Als "Weichmacher" werden flüssige oder gelöste Substanzen bezeichnet, welche in einem ausgehärteten Polymer nicht chemisch eingebunden sind und typischerweise eine weichmachende Wirkung auf das Polymer ausüben.

Das Aldehydgemisch der erfindungsgemässen Verwendung stellt insbesondere ein Umsetzungsprodukt einer Formylierung von mindestens einem Alkylbenzol der Formel 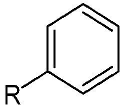 dar, wobei R die bereits beschriebenen Bedeutungen aufweist.

Dabei wurde das Umsetzungsprodukt insbesondere nicht oder nur soweit aufgereinigt, dass sowohl der Hauptbestandteil - die beschriebenen Aldehyde der Formel (I) - durch das Aufreinigungsverfahren, wie beispielsweise Extraktion oder insbesondere Destillation bzw. Fraktionierung, als auch der Nebenbestandteil - die beschriebenen nicht der Formel (I) entsprechenden Alkylbenzol-Verbindungen, welche Nebenprodukte der Formylierung darstellen - weitestgehend im Umsetzungsprodukt bleiben und nicht voneinander getrennt werden. Ein solches Umsetzungsprodukt wird im Folgenden auch als "Rohprodukt" bezeichnet.

Bevorzugt wird die Formylierung mit Kohlenmonoxid (CO) als Formylierungsreagens duchgeführt.

Bevorzugt wird die Formylierung des Alkylbenzols mit Kohlenmonoxid in Gegenwart von Flusssäure und Bortrifluorid (HF-BF₃ bzw. HBF₄) als Säurekatalysator durchgeführt. Dieses Verfahren ist besonders vorteilhaft, da es unter milden Bedingungen durchführbar ist und dadurch besonders selektiv verläuft. Mit seinem hohen Dampfdruck kann der Katalysator einfach abgetrennt und wiederverwendet werden, womit eine aufwendige Reinigung und Abfallentsorgung wie bei anderen Katalysatorsystemen entfällt.

Bevorzugt erfolgt die Umsetzung bei einem Kohlenmonoxid-Partialdruck von 5 bis 50 bar, insbesondere 10 bis 30 bar, und einer Temperatur von -50 bis +20°C, insbesondere -30 bis 0°C.

Das molare Verhältnis Alkylbenzol zu HF zu BF₃ liegt bevorzugt im Bereich von 1 zu (2.5 bis 30) zu (1 bis 5), insbesondere 1 zu (3 bis 25) zu (1.1 bis 3). Bevorzugt wird das Umsetzungsprodukt auf Eiswasser gegeben und mittels eines Lösemittels, insbesondere eines Alkans wie Hexan oder Heptan, extrahiert. Das Extrakt wird anschliessend bevorzugt durch Waschen mit Wasser oder insbesondere wässriger Base, beispielsweise einer wässrigen Natriumhydroxid-Lösung, von Säurespuren befreit und anschliessend das Lösemittel und gegebenenfalls vorhandene weitere niedermolekulare Bestandteile destillativ aus der Reaktionsmischung entfernt.

Gegebenenfalls wird das so erhaltene Rohprodukt mit einer geeigneten Methode geklärt oder farblich aufgehellt, beispielsweise mittels Filtration oder Adsorption.

Auf eine weitere Aufreinigung des so erhaltenen Rohprodukts, insbesondere eine Destillation bzw. Fraktionierung der Aldehyde der Formel (I), wird verzichtet, was in Bezug auf Produktionsaufwand und Kosten besonders vorteilhaft ist.

Bevorzugt enthält das Aldehydgemisch einen Gehalt an Aldehyden der Formel (I) im Bereich von 80 bis 92 Gewichts-% und einen Gehalt an nicht der Formel (I) entsprechenden Alkylbenzol-Verbindungen von 8 bis 20 Gewichts-%.

Bevorzugt ist das Aldehydgemisch frei von niedermolekularen Bestandteilen und daher geruchlos. Insbesondere ist das Aldehydgemisch frei von Lösemitteln, Benzaldehyd und von C₁₋₅-Alkylbenzaldehyden.

Bevorzugt ist das Aldehydgemisch hellfarbig.

Insbesondere hat es eine Gardner-Farbzahl nach DIN ISO 4630 von höchstens 12, bevorzugt höchstens 10. Damit erhaltene latente Härter eignen sich auch für hellfarbige Polyurethanzusammensetzungen.

Bevorzugt weist das mit der erfindungsgemässen Verwendung zu blockierende Amin mindestens eine primäre oder sekundäre Aminogruppe auf.

Besonders bevorzugt weist das zu blockierende Amin mindestens eine primäre oder sekundäre Aminogruppe und zusätzlich mindestens eine Reaktivgruppe ausgewählt aus primärer Aminogruppe, sekundärer Aminogruppe, Hydroxylgruppe und Silangruppe auf.

Ein solches Amin ist geeignet als latenter Härter und/oder Vernetzer und/oder Haftvermittler für Verbindungen mit Reaktivgruppen wie insbesondere Isocyanatgruppen. In blockierter Form reagiert es unter Ausschluss von Feuchtigkeit nicht oder nur sehr langsam mit solchen Verbindungen, während es bei Kontakt mit Feuchtigkeit unter Hydrolyse reagieren kann.

Geeignete Amine zum Blockieren sind insbesondere ausgewählt aus der Gruppe bestehend aus primären aliphatischen Diaminen, primären aromatischen Diaminen, primären aliphatischen Triaminen, aliphatischen Diaminen mit einer primären und einer sekundären Aminogruppe, aliphatischen Polyaminen mit zwei primären und einer sekundären Aminogruppe, Aminoalkoholen, Dialkanolaminen, Aminosilanen und Alkanolaminosilanen.

Geeignete primäre aliphatische Diamine sind insbesondere 1,2-Ethandiamin, 1,2-Propandiamin, 1,3-Propandiamin, 1,4-Butandiamin, 1,3-Butandiamin, 2-Methyl-1,2-propandiamin, 1,3-Pentandiamin, 1,5-Pentandiamin, 2,2-Dimethyl-1,3-propandiamin, 1,6-Hexandiamin, 1,5-Diamino-2-methylpentan, 1,7-Heptandiamin, 1,8-Octandiamin, 2,5-Dimethyl-1,6-hexandiamin, 1,9-Nonandiamin, 2,2(4),4-Trimethyl-1,6-hexandiamin, 1,10-Decandiamin, 1,11-Undecandiamin, 2-Butyl-2-ethyl-1,5-pentandiamin, 1,12-Dodecandiamin, 1,2-Cyclohexandiamin, 1,3-Cyclohexandiamin, 1,4-Cyclohexandiamin, 1-Amino-3-aminomethyl-3,5,5-trimethylcyclohexan, 4(2)-Methyl-1,3-cyclohexandiamin, 1,3-Bis(aminomethyl)-cyclohexan, 1,4-Bis(aminomethyl)cyclohexan, Bis(4-aminocyclohexyl)methan, Bis(4-amino-3-methylcyclohexyl)methan, Bis(4-amino-3-ethylcyclohexyl)methan, Bis-(4-amino-3,5-dimethylcyclohexyl)methan, Bis(4-amino-3-ethyl-5-methylcyclohexyl)methan, 2,5(2,6)-Bis(aminomethyl)bicyclo[2.2.1]heptan, 3(4),8(9)-Bis(aminomethyl)tricyclo[5.2.1.0^{2,6}]decan, 1,3-Bis(aminomethyl)-benzol, 1,4-Bis(aminomethyl)benzol, 3-Oxa-1,5-pentandiamin, 3,6-Dioxaoctan-1,8-diamin, 4,7-Dioxadecan-1,10-diamin, 4,7-Dioxadecan-2,9-diamin, 4,9-Di-oxadodecan-1,12-diamin, 5,8-Dioxadodecan-3,10-diamin, 4,7,10-Trioxatri-decan-1,13-diamin, α,ω-Polyoxypropylendiamin mit einem mittleren Molekulargewicht im Bereich von 200 bis 4'000 g/mol, insbesondere die Jeffamine^{®}-Typen D-230, D-400, XTJ-582, D-2000, XTJ-578, D-4000 (alle von Huntsman), α,ω-Polyoxypropylenpolyoxyethylendiamin, insbesondere die Jeffamine^{®}-Typen ED-600, ED-900, ED-2003, HK-511 (alle von Huntsman), α,ω-Polyoxypro-pylenpolyoxy-1,4-butylendiamin, insbesondere die Jeffamine^{®}-Typen THF-100, THF-140, THF-230, XTJ-533 oder XTJ-536 (alle von Huntsman), α,ω-Polyoxy-propylenpolyoxy-1,2-butylendiamin, insbesondere die Jeffamine^{®}-Typen XTJ-568 oder XTJ-569 (beide von Huntsman) oder α,ω-Polyoxy-1,2-butylendiamin, insbesondere Jeffamine^{®} XTJ-523 (von Huntsman).

Geeignete primäre aromatische Diamine sind insbesondere 1,3-Phenylendiamin, 1,4-Phenylendiamin, 4(2)-Methyl-1,3-phenylendiamin (TDA), 3,5-Diethyl-2,4(6)-toluylendiamin (DETDA) oder 4,4'-Diaminodiphenylmethan (MDA).

Geeignete primäre aliphatische Triamine sind insbesondere 1,3,6-Triaminohexan, 1,4,8-Triaminooctan, 4-Aminomethyl-1,8-octandiamin, 5-Aminomethyl-1,8-octandiamin, 1,6,11-Triaminoundecan, 1,3,5-Triaminocyclohexan, 1,3,5-Tris-(aminomethyl)cyclohexan, 1,3,5-Tris(aminomethyl)benzol, Trimethylolpropan- oder Glycerin-gestartetes Tris(ω-polyoxypropylenamin) mit einem mittleren Molekulargewicht im Bereich von 380 bis 6'000 g/mol, insbesondere die Jeffamine^{®}-Typen T-403, T-3000 oder T-5000 (alle von Huntsman), oder Trimethylolpropan-gestartetes Tris(ω-polyoxypropylen-polyoxy-1,2-butylenamin), insbesondere Jeffamine^{®} XTJ-566 (von Huntsman).

Geeignete aliphatische Diamine mit einer primären und einer sekundären Aminogruppe sind insbesondere N-Methyl-1,2-ethandiamin, N-Ethyl-1,2-ethandiamin, N-Butyl-1,2-ethandiamin, N-Hexyl-1,2-ethandiamin, N-(2-Ethylhexyl)-1,2-ethandiamin, N-Cyclohexyl-1,2-ethandiamin, N-Benzyl-1,2-ethandiamin, 4-Aminomethylpiperidin, 3-(4-Aminobutyl)piperidin, N-(2-Aminoethyl)piperazin, N-(2-Aminopropyl)piperazin, N-Benzyl-1,2-propandiamin, N, Benzyl-1,3-propandiamin, N-Methyl-1,3-propandiamin, N-Ethyl-1,3-propandiamin, N-Butyl-1,3-propandiamin, N-Hexyl-1,3-propandiamin, N-(2-Ethylhexyl)-1,3-propandiamin, N-Dodecyl-1,3-propandiamin, N-Cyclohexyl-1,3-propandiamin, 3-Methylamino-1-pentylamin, 3-Ethylamino-1-pentylamin, 3-Butylamino-1-pentylamin, 3-Hexyl-amino-1-pentylamin, 3-(2-Ethylhexyl)amino-1-pentylamin, 3-Dodecylamino-1-pentylamin, 3-Cyclohexylamino-1-pentylamin, Fettdiamine wie N-Cocoalkyl-1,3-propandiamin, N-Oleyl-1,3-propandiamin, N-Soyaalkyl-1,3-propandiamin, N-Talgalkyl-1,3-propandiamin oder N-(C₁₆₋₂₂-Alkyl)-1,3-propandiamin, wie sie beispielsweise unter dem Handelsnamen Duomeen^{®} von Akzo Nobel erhältlich sind, oder Produkte aus der Michael-artigen Addition von aliphatischen primären Diaminen mit Acrylnitril, Malein- oder Fumarsäurediestern, Citraconsäurediestern, (Meth)acrylsäureestern, (Meth)acrylsäureamiden oder Itaconsäurediestern, umgesetzt im Molverhältnis 1:1.

Geeignete aliphatische Polyamine mit zwei primären und einer sekundären Aminogruppe sind insbesondere Bis(hexamethylen)triamin (BHMT), Diethylentriamin (DETA), Dipropylentriamin (DPTA), N-(2-Aminoethyl)-1,3-propandiamin (N3-Amin), N3-(3-Aminopentyl)-1,3-pentandiamin oder N5-(3-Amino-1-ethylpropyl)-2-methyl-1 , 5-pentandiamin.

Geeignete Aminoalkohole sind insbesondere 2-Aminoethanol, 2-Amino-1-propanol, 1-Amino-2-propanol, 3-Amino-1-propanol, 4-Amino-1-butanol, 4-Amino-2-butanol, 2-Amino-2-methylpropanol, 5-Amino-1-pentanol, 6-Amino-1-hexanol, 7-Amino-1-heptanol, 8-Amino-1-octanol, 10-Amino-1-decanol, 12-Amino-1-dodecanol oder höhere Homologe davon, 4-(2-Aminoethyl)-2-hydroxyethylbenzol, 3-Aminomethyl-3,5,5-trimethylcyclohexanol, eine primäre Aminogruppe tragende Derivate von Glykolen wie Diethylenglykol, Dipropylenglykol, Dibutylenglykol oder höhere Oligomere oder Polymere dieser Glykole, insbesondere 2-(2-Aminoethoxy)ethanol, 2-(2-(2-Aminoethoxy)ethoxy)ethanol oder α-(2-Hydroxymethylethyl)-ω-(2-aminomethylethoxy)-poly(oxy(methyl-1,2-ethandiyl), 3-(2-Hydroxyethoxy)propylamin, 3-(2-(2-Hydroxyethoxy)ethoxy)propylamin oder 3-(6-Hydroxyhexyloxy)propylamin, N-Methylethanolamin, N-Ethylethanolamin, N-n-Propylethanolamin, N-Isopropylethanolamin, N-n-Butylethanolamin, N-Isobutylethanolamin, N-2-Butylethanolamin, N-tert.Butylethanolamin, N-Hexylethanolamin, N-Isohexylethanolamin oder N-(2-Ethylhexyl)-ethanolamin, sowie N-(2-Aminoethyl)ethanolamin oder Umsetzungsprodukte von primären aliphatischen Diaminen wie den vorgängig genannten mit Monoepoxiden im Molverhältnis 1:1, insbesondere das Umsetzungsprodukt einer überstöchiometischen Menge von 1,2-Propandiamin oder 2-Methyl-1,5-pentandiamin mit Kresylglyciylether und nachfolgender Entfernung von nicht umgesetztem 1,2-Propandiamin bzw. 2-Methyl-1,5-pentandiamin; weiterhin mehrwertige Aminoalkohole, insbesondere N,N'-Dialkoxylierungsprodukte der genannten primären aliphatischen Diamine, wie beispielsweise Jeffamine^{®} C-346 (von Huntsman), oder N,N'-Diaddukte der genannten primären aliphatischen Diamine mit Monoglycidylethern wie insbesondere Phenylglyciylether, Kresylglyciylether, tert.Butylphenylglyciylether, Cardanolglyciylether oder 2-Ethylhexylglyciylether, oder α,ω-Diaddukte von Diglycidylethern, wie insbesondere Butandioldiglycidylether, Hexandioldiglycidylether, Dipropylenglycoldiglycidylether, Polypropylenglycoldiglyciylether, Bisphenol-A-Diglyciylether oder Bisphenol-F-Diglycidylether, mit Monoaminen, wie insbesondere Isopropylamin, n-Butylamin, Isobutylamin, 2-Butylamin, tert.Butylamin, n-Hexylamin, Isohexylamin, 2-Ethylhexylamin oder Benzylamin.

Geeignete Dialkanolamine sind insbesondere Diethanolamin, Diisopropanolamin oder 2-Amino-2-ethyl-1,3-propandiol.

Geeignete Aminosilane sind insbesondere 3-Aminopropyltrimethoxysilan, 3-Aminopropyltriethoxysilan oder 3-Aminopropyldimethoxymethylsilan.

Geeignete Alkanolaminosilane sind insbesondere Alkoxylate bzw. Addukte der genannten Aminosilane mit Epoxiden, wie insbesondere Ethylenoxid, Propylenoxid, Phenylglycidylether, Kresylglycidylether oder einem Alkylglycidylether, oder einem Epoxysilan, wie insbesondere 3-Glycidoxypropyltrimethoxysilan oder 3-Glycidoxypropyltriethoxysilan.

Bevorzugte Amine zum Blockieren sind primäre aliphatische Diamine, primäre aliphatische Triamine oder Dialkanolamine.

Besonders bevorzugte Amine zum Blockieren sind ausgewählt aus der Gruppe bestehend aus Diethanolamin, 1,6-Hexandiamin, 1-Amino-3-aminomethyl-3,5,5-trimethylcyclohexan, 4(2)-Methyl-1,3-cyclohexandiamin, 1,3-Bis(aminomethyl)cyclohexan, 1,4-Bis(aminomethyl)cyclohexan, 1,3-Bis(aminomethyl)-benzol, 1,2-Cyclohexandiamin, 1,3-Cyclohexandiamin, 1,4-Cyclohexandiamin, Bis(4-aminocyclohexyl)methan, 2,5(2,6)-Bis(aminomethyl)bicyclo[2.2.1]heptan, 3(4),8(9)-Bis(aminomethyl)tricyclo[5.2.1.0^{2,6}]decan, α,ω-Polyoxypropylendiamin mit einem mittleren Molekulargewicht im Bereich von 200 bis 4'000 g/mol, insbesondere die Jeffamine^{®}-Typen D-230, D-400, XTJ-582, D-2000, XTJ-578, D-4000 (alle von Huntsman) und Trimethylolpropan- oder Glycerin-gestartetes Tris(ω-polyoxypropylenamin) mit einem mittleren Molekulargewicht im Bereich von 380 bis 6'000 g/mol, insbesondere die Jeffamine^{®}-Typen T-403, T-3000 oder T-5000 (alle von Huntsman).

Davon bevorzugt ist Diethanolamin, 1,6-Hexandiamin, 1-Amino-3-aminomethyl-3,5,5-trimethylcyclohexan, 1,3-Bis(aminomethyl)benzol, α,ω-Polyoxypropylendiamin mit einem mittleren Molekulargewicht von ungefähr 240 g/mol oder Trimethylolpropan-gestartetes Tris(ω-polyoxypropylenamin) mit einem mittleren Molekulargewicht von ungefähr 440 g/mol.

Diese Amine sind einfach zugänglich, in blockierter Form niedrigviskos und ermöglichen Polyurethanzusammensetzungen mit guter Lagerstabilität, guter Verarbeitbarkeit, schneller Aushärtung bei moderater Offenzeit und ausgezeichneten mechanischen Eigenschaften, insbesondere überraschend hoher Festigkeit bei hoher Dehnbarkeit.

In der erfindungsgemässen Verwendung des Aldehydgemisches wird das Aldehydgemisch mit dem zu blockierenden Amin bevorzugt so umgesetzt, dass
- das Aldehydgemisch mit dem Amin zu einer Reaktionsmischung vereint wird, gegebenenfalls unter Einsatz eines Lösemittels, wobei die Aldehydgruppen gegenüber den primären und sekundären Aminogruppen bevorzugt stöchiometrisch oder im stöchiometrischen Überschuss vorhanden sind, und
- das bei der Reaktion entstehende Kondensationswasser und gegebenenfalls eingesetztes Lösemittel während oder nach der Vereinigung mit einer geeigneten Methode aus der Reaktionsmischung entfernt werden,
gegebenenfalls unter deren Erwärmen und/oder Anlegen von Vakuum. Bevorzugt wird das Kondensationswasser mittels Anlegen von Vakuum aus der erwärmten Reaktionsmischung entfernt, ohne dass dabei Lösemittel eingesetzt werden.

Die Umsetzung wird bevorzugt bei einer Temperatur im Bereich von 20 bis 120°C, insbesondere bei 40 bis 100°C, durchgeführt.

Gegebenenfalls wird bei der Umsetzung ein Katalysator eingesetzt, insbesondere ein Säure-Katalysator.

Ein weiterer Gegenstand der Erfindung ist ein blockiertes Amin erhalten aus der erfindungsgemässen Verwendung des beschriebenen Aldehydgemisches.

Das blockierte Amin weist insbesondere funktionelle Gruppen der Formeln (II) und/oder (III) auf, 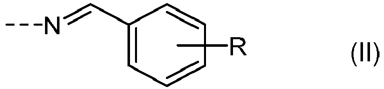 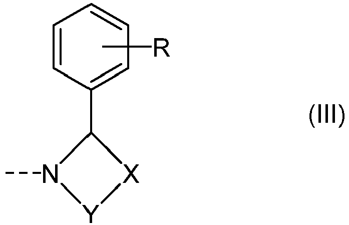 wobei
X für O oder S oder NR⁰ steht, wobei R° für einen einwertigen organischen Rest mit 1 bis 18 C-Atomen steht,
Y für einen 1,2-Ethylen- oder 1,3-Propylen-Rest, welcher gegebenenfalls substituiert ist, steht,
und R die bereits beschriebenen Bedeutungen aufweist.

Bevorzugt steht R in der funktionellen Gruppe der Formel (II) oder (III) in meta- oder para-Stellung, insbesondere in para-Stellung.

Die funktionelle Gruppe der Formel (II) entsteht aus der Kondensationsreaktion einer primären Aminogruppe mit einem Aldehyd der Formel (I). Sie stellt eine Aldiminogruppe dar.

Die funktionelle Gruppe der Formel (III) entsteht aus der Kondensationsreaktion einer Gruppierung der Formel ---NH-Y-XH mit einem Aldehyd der Formel (I), wobei Y und X die bereits genannten Bedeutungen aufweisen.

Bevorzugt steht R⁰ für einen Alkyl-, Cycloalkyl- oder Arylalkyl-Rest mit 1 bis 18 C-Atomen, der gegebenenfalls eine oder zwei Gruppen ausgewählt aus Carbonsäureester-, Nitril-, Nitro-, Phosphonsäureester-, Sulfon- und Sulfonsäureester-Gruppen trägt.

Bevorzugt steht X für O.

Bevorzugt sind NH und XH durch zwei C-Atome voneinander getrennt.

Eine solche funktionelle Gruppe der Formel (III) stellt eine Oxazolidino-Gruppe dar. Sie ist besonders reaktiv bei der Verwendung als latenter Härter in Polyurethanzusammensetzungen.

Bevorzugt steht Y für einen 1,2-Ethylen- oder 1,3-Propylen- oder 1,2-Propylen-Rest oder für einen gegebenenfalls substituierten und gegebenenfalls eine Silangruppe aufweisenden 3-Alkyloxy-1,2-propylen- oder 3-Aryloxy-1,2-propylen-Rest steht, oder kann im Fall von n = 1 und X = O auch für einen Rest der Formel 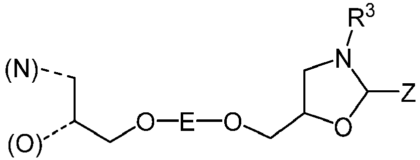 stehen, wobei E für einen zweiwertigen, gegebenenfalls eine oder mehrere Ether-, Ester- oder Glycidoxy-Gruppen tragenden Kohlenwasserstoff-Rest mit 6 bis 20 C-Atomen steht, und R³ für einen gegebenenfalls eine oder mehrere Ether-Gruppen tragenden Alkyl-, Cycloalkyl- oder Aralkyl-Rest mit 1 bis 35 C-Atomen steht.

Besonders bevorzugt stehen X für O und Y für 1,2-Ethylen oder für einen gegebenenfalls substituierten 3-Alkyloxy-1,2-propylen- oder 3-Aryloxy-1,2-propylen-Rest.

Bevorzugt steht R³ für Methyl, Ethyl, n-Propyl, Isopropyl, n-Butyl, Isobutyl, 2-Butyl, tert.Butyl, n-Hexyl, Isohexyl, 2-Ethylhexyl, Dodecyl, Benzyl oder einen Methoxy-terminierten Polyoxypropylen-Rest, welcher auch Oxyethylen-Anteile enthalten kann.

Bevorzugt steht E für 4,4'-Methylendiphenyl, 4,4'-(2,2-Propylen)diphenyl, 1,2- oder 1,3- oder 1,4-Phenyl, 1,4-Butylen, 1,6-Hexylen oder α,ω-Polyoxypropylen mit einem mittleren Molekulargewicht im Bereich von 96 bis 1'000 g/mol.

Das blockierte Amin kann vorteilhaft verwendet werden als latenter Härter und/oder Vernetzer und/oder Haftvermittler.

Für die Verwendung als Haftvermittler enthält das blockierte Amin bevorzugt mindestens eine Silangruppe. Der Haftvermittler ist insbesondere nützlich für den Einsatz in Isocyanat- und/oder Silangruppen-haltigen Zusammensetzungen.

Bevorzugt wird das blockierte Amin verwendet als latenter Härter für Zusammensetzungen, welche Reaktivgruppen enthalten, die mit Aminwasserstoffen reagieren können, wie insbesondere Isocyanatgruppen oder Epoxidgruppen. Für diese Verwendung enthält das blockierte Amin neben funktionellen Gruppen der Formel (II) und/oder (III) bevorzugt mindestens eine weitere Reaktivgruppe ausgewählt aus der Gruppe bestehend aus funktionellen Gruppen der Formeln (II) oder (III), Hydroxylgruppen, Mercaptogruppen, primären Aminogruppen und sekundären Aminogruppen.

Bevorzugt sind latente Härter, welche zwei oder drei funktionelle Gruppen der Formel (II) aufweisen, also Dialdimine oder Trialdimine darstellen.

Weiterhin bevorzugt sind latente Härter, welche eine funktionelle Gruppe der Formel (II) und eine Hydroxylgruppe oder primäre Aminogruppe oder sekundäre Aminogruppe, insbesondere eine Hydroxylgruppe, aufweisen, also Hydroxyaldimine oder Aminoaldimine darstellen.

Weiterhin bevorzugt sind latente Härter, welche eine funktionelle Gruppe der Formel (II) und eine funktionelle Gruppe der Formel (III) aufweisen und insbesondere Aldimino-Oxazolidine darstellen.

Weiterhin bevorzugt sind latente Härter, welche zwei funktionelle Gruppen der Formel (III) aufweisen und insbesondere Bisoxazolidine darstellen.

Weiterhin bevorzugt sind latente Härter, welche eine funktionelle Gruppe der Formel (III) und eine Hydroxylgruppe aufweisen und insbesondere Hydroxyoxazolidine darstellen.

Besonders bevorzugt sind Dialdimine, Trialdimine, Hydroxyaldimine, Bisoxazolidine und Hydroxyoxazolidine.

Am meisten bevorzugt sind Dialdimine und Trialdimine, insbesondere Dialdimine.

Latente Härter, welche eine freie OH- oder NH-Gruppe enthalten, reagieren beim Vermischen mit Polyisocyanaten zu Isocyanatgruppen-haltigen Umsetzungsprodukten, wobei diese ihrerseits latente Härter darstellen und bei Zutritt von Feuchtigkeit als Vernetzer wirken.

Für den Fall, dass Diethanolamin als zu blockierendes Amin eingesetzt wird, wird ein Hydroxyoxazolidin der Formel 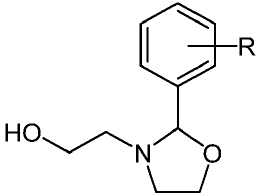 erhalten. Solche Hydroxyoxazolidine sind besonders geeignet für die Umsetzung mit einem Diisocyanat oder mit einem Carbonat zu einem Bisoxazolidin der Formel 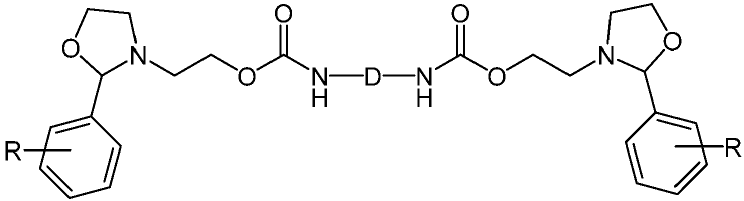 oder 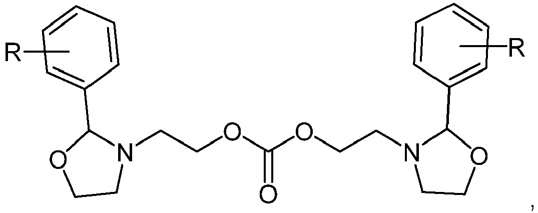 wobei D für einen zweiwertigen Kohlenwasserstoff-Rest mit 6 bis 15 C-Atomen, insbesondere 1,6-Hexamethylen oder (1,5,5-Trimethylcyclohexan-1-yl)methan-1,3 oder 4(2)-Methyl-1,3-phenylen, steht und R die bereits beschriebenen Bedeutungen aufweist.

Die latenten Härter sind besonders geeignet für Isocyanatgruppen-haltige Zusammensetzungen, insbesondere Polyurethanzusammensetzungen.

Der latente Härter weist neben funktionellen Gruppen der Formel (II) und/oder (III) Anteile zusätzlicher funktioneller Gruppen aus Kondensationsreaktionen der nicht der Formel (I) entsprechenden Alkylbenzol-Verbindungen mit Aminogruppen. Überraschenderweise beeinträchtigen diese Anteile die Verwendung des latenten Härters nicht, sondern ergeben sogar Vorteile. So sind die latenten Härter niedrigviskoser und weisen eine tiefere Kristallisationstemperatur auf als entsprechende latente Härter, zu deren Herstellung ein aufgereinigtes Blockierungsmittel in Form von reinen Aldehyden der Formel (I) verwendet wurde, und ergeben in Polyurethanzusammensetzungen sogar höhere Festigkeiten, ohne dass dabei Einbussen bei der Lagerstabilität auftreten.

Ein weiterer Gegenstand der Erfindung ist eine Isocyanatgruppen-haltige Zusammensetzung enthaltend mindestens ein blockiertes Amin erhalten aus der beschriebenen Verwendung.

Bevorzugt enthält die Isocyanatgruppen-haltige Zusammensetzung
- mindestens ein Polyisocyanat und/oder mindestens ein Isocyanatgruppen-haltiges Polyurethanpolymer, und
- mindestens ein Polyaldimin der Formel (IV),
wobei 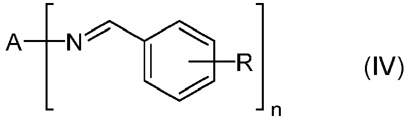
n für 2 oder 3 steht,
A für einen n-wertigen organischen Rest mit einem Molekulargewicht im Bereich von 28 bis 10'000 g/mol steht,
und R die bereits beschriebenen Bedeutungen aufweist.

Bevorzugt steht R in meta- oder para-Stellung, insbesondere in para-Stellung.

Bevorzugt steht A für einen n-wertigen Kohlenwasserstoff-Rest mit einem Molekulargewicht im Bereich von 28 bis 6'000 g/mol, der gegebenenfalls eine oder mehrere Gruppen ausgewählt aus Ether-, Ester-, Carbonat-, Urethan- und Harnstoff-Gruppen aufweist.

Bevorzugt steht n für 2.

Bevorzugt steht A für einen n-wertigen aliphatischen, cycloaliphatischen oder arylaliphatischen, gegebenenfalls Ether-Sauerstoff aufweisendenen Kohlenwasserstoff-Rest mit einem Molekulargewicht im Bereich von 28 bis 6'000 g/mol. Solche auf aliphatischen Aminen basierende Polyaldimine sind toxikologisch besonders vorteilhaft.

Besonders bevorzugt steht A für 1,6-Hexylen, (1,5,5-Trimethylcyclohexan-1-yl)methan-1,3, 4(2)-Methyl-1,3-cyclohexylen, 1,3-Cyclohexylen-bis(methylen), 1,4-Cyclohexylen-bis(methylen), 1,3-Phenylen-bis(methylen), 1,2-Cyclohexylen, 1,3-Cyclohexylen, 1,4-Cyclohexylen, Methylenbis(2-methylcyclohexan-4-yl), (Bicyclo[2.2.1]heptan-2,5(2,6)-diyl)dimethylen, (Tricyclo[5.2.1.0^{2,6}]decan-3(4),8(9)-diyl)dimethylen, α,ω-Polyoxypropylen mit einem mittleren Molekulargewicht im Bereich von 170 bis 4'000 g/mol und Trimethylolpropan- oder Glycerin-gestartetes Tris(ω-polyoxypropylen) mit einem mittleren Molekulargewicht im Bereich von 330 bis 6'000 g/mol.

Insbesondere ist A ausgewählt aus der Gruppe bestehend aus 1,6-Hexylen, (1,5,5-Trimethylcyclohexan-1-yl)methan-1,3, 1,3-Cyclohexylen-bis(methylen), 1,3-Phenylen-bis(methylen), α,ω-Polyoxypropylen mit einem mittleren Molekulargewicht im Bereich von 170 bis 470 g/mol und Trimethylolpropan-gestartetes Tris(co-polyoxypropylen) mit einem mittleren Molekulargewicht im Bereich von 330 bis 450 g/mol.

Solche Polyaldimine sind besonders gut zugänglich, bei Raumtemperatur niedrigviskos und ermöglichen Zusammensetzungen mit guten mechanischen Eigenschaften, insbesondere hoher Festigkeit und hoher Dehnbarkeit.

Als Polyisocyanat geeignet ist insbesondere ein im Handel erhältliches Polyisocyanat, insbesondere
- aromatische Di- oder Triisocyanate, bevorzugt 4,4'- oder 2,4'- oder 2,2'-Diphenylmethandiisocyanat oder beliebigen Gemischen dieser Isomeren (MDI), 2,4- oder 2,6-Toluylendiisocyanat oder beliebige Gemische dieser Isomeren (TDI), Gemische aus MDI und MDI-Homologen (polymeres MDI oder PMDI), 1,3- oder 1,4-Phenylendiisocyanat, 2,3,5,6-Tetramethyl-1,4-diisocyanatobenzol, Naphthalin-1,5-diisocyanat (NDI), 3,3'-Dimethyl-4,4'-diisocyanatodiphenyl (TODI), Dianisidindiisocyanat (DADI), Tris-(4-isocyanatophenyl)methan oder Tris-(4-isocyanatophenyl)thiophosphat; bevorzugt MDI oder TDI;
- aliphatische, cycloaliphatische oder arylaliphatische Di- oder Triisocyanate, bevorzugt 1,4-Tetramethylendiisocyanat, 2-Methylpentamethylen-1,5-diisocyanat, 1,6-Hexamethylendiisocyanat (HDI), 2,2,4- und/oder 2,4,4-Trimethyl-1,6-hexamethylendiisocyanat (TMDI), 1,10-Decamethylendiisocyanat, 1,12-Dodecamethylendiisocyanat, Lysin- oder Lysinesterdiisocyanat, Cyclohexan-1,3- oder -1,4-diisocyanat, 1-Methyl-2,4- und/oder -2,6-diisocyanatocyclohexan \(H₆TDI), 1-Isocyanato-3,3,5-trimethyl-5-isocyanatomethylcyclohexan (IPDI), Perhydro-2,4'- und/oder -4,4'-diphenylmethandiisocyanat (H₁₂MDI), 1,3- oder 1,4-Bis-(isocyanatomethyl)cyclohexan, m- oder p-Xylylendiisocyanat, Tetramethyl-1,3- oder -1,4-xylylendiisocyanat, 1,3,5-Tris-(isocyanatomethyl)benzol, Bis-(1-Isocyanato-1-methylethyl)naphthalin, Dimer- oder Trimerfettsäureisocyanate wie insbesondere 3,6-Bis-(9-isocyanatononyl)-4,5-di(1-heptenyl)cyclohexen (Dimeryldiisocyanat); bevorzugt H₁₂MDI oder HDI oder IPDI;.
- Oligomere oder Derivate der genannten Di- oder Triisocyanate, insbesondere abgeleitet von HDI, IPDI, MDI oder TDI, insbesondere Oligomere enthaltend Uretdion- oder Isocyanurat- oder Iminooxadiazindion-Gruppen oder verschiedene dieser Gruppen; oder zwei- oder mehrwertige Derivate enthaltend Ester- oder Harnstoff- oder Urethan- oder Biuret- oder Allophanat- oder Carbodiimid- oder Uretonimin- oder Oxadiazintrion-Gruppen oder verschiedene dieser Gruppen. Solche Polyisocyanate stellen in der Praxis üblicherweise Gemische von Substanzen mit unterschiedlichen Oligomerisierungsgraden und/oder chemischen Strukturen dar. Insbesondere weisen sie eine mittlere NCO-Funktionalität von 2.1 bis 4.0 auf.

Als Polyisocyanat bevorzugt sind aliphatische, cycloaliphatische oder aromatiche Diisocyanate, insbesondere HDI, IPDI, H₁₂MDI, TDI oder MDI, insbesondere HDI, IPDI, TDI oder MDI.

Ein geeignetes Isocyanatgruppen-haltiges Polyurethanpolymer wird insbesondere erhalten aus der Umsetzung von mindestens einem Polyol mit einer überstöchiometrischen Menge von mindestens einem Polyisocyanat. Die Umsetzung wird bevorzugt unter Feuchtigkeitsausschluss bei einer Temperatur im Bereich von 50 bis 160°C durchgeführt, gegebenenfalls in Anwesenheit geeigneter Katalysatoren. Das NCO/OH-Verhältnis liegt bevorzugt im Bereich von 1.3/1 bis 2.5/1. Das nach der Umsetzung der OH-Gruppen im Reaktionsgemisch verbleibende Polyisocyanat, insbesondere monomeres Diisocyanat, kann entfernt werden, insbesondere mittels Destillation, was im Fall eines hohen NCO/OH-Verhältnisses bevorzugt ist. Das erhaltene Polyurethanpolymer weist bevorzugt einen Gehalt an freien Isocyanatgruppen im Bereich von 0.5 bis 10 Gewichts-%, insbesondere 1 bis 5 Gewichts-%, besonders bevorzugt 1 bis 3 Gewichts-%, auf. Gegebenenfalls kann das Polyurethanpolymer unter Mitverwendung von Weichmachern oder Lösemitteln hergestellt werden, wobei die verwendeten Weichmacher oder Lösemittel keine gegenüber Isocyanaten reaktive Gruppen enthalten.

Als Polyisocyanat zur Herstellung eines Isocyanatgruppen-haltigen Polyurethanpolymers bevorzugt sind die bereits genannten Polyisocyanate, insbesondere die Diisocyanate, bevorzugt MDI, TDI, IPDI oder HDI.

Geeignete Polyole sind handelsübliche Polyole oder Mischungen davon, insbesondere
- Polyetherpolyole, insbesondere Polyoxyalkylendiole und/oder Polyoxyalkylentriole, insbesondere Polymerisationsprodukte von Ethylenoxid oder 1,2-Propylenoxid oder 1,2- oder 2,3-Butylenoxid oder Oxetan oder Tetrahydrofuran oder Mischungen davon, wobei diese mit Hilfe eines Startermoleküls mit zwei oder mehreren aktiven Wasserstoffatomen polymerisiert sein können, insbesondere einem Startermolekül wie Wasser, Ammoniak oder einer Verbindung mit mehreren OH- oder NH-Gruppen wie beispielsweise 1,2-Ethandiol, 1,2- oder 1,3-Propandiol, Neopentylglykol, Diethylenglykol, Triethylenglykol, die isomeren Dipropylenglykole oder Tripropylenglykole, die isomeren Butandiole, Pentandiole, Hexandiole, Heptandiole, Octandiole, Nonandiole, Decandiole, Undecandiole, 1,3- oder 1,4-Cyclohexandimethanol, Bisphenol A, hydriertes Bisphenol A, 1,1,1-Trimethylolethan, 1,1,1-Trimethylolpropan, Glycerin oder Anilin, oder Mischungen der vorgenannten Verbindungen. Ebenfalls geeignet sind Polyetherpolyole mit darin dispergierten Polymerpartikeln, insbesondere solche mit Styrol-Acrylnitril-Partikeln (SAN) oder Polyharnstoff- bzw. Polyhydrazodicarbonamid-Partikeln (PHD). Bevorzugte Polyetherpolyole sind Polyoxypropylendiole oder Polyoxypropylentriole, oder sogenannte Ethylenoxid-terminierte (EO-endcapped) Polyoxypropylendiole oder -triole. Letztere sind Polyoxyethylen-polyoxypropylen-Mischpolyole, die insbesondere dadurch erhalten werden, dass Polyoxypropylendiole oder -triole nach Abschluss der Polypropoxylierungsreaktion mit Ethylenoxid weiter alkoxyliert werden und dadurch schliesslich primäre Hydroxylgruppen aufweisen.
   Bevorzugte Polyetherpolyole weisen einen Ungesättigtheitsgrad von weniger als 0.02 mEq/g, insbesondere weniger als 0.01 mEq/g auf.
- Polyesterpolyole, auch Oligoesterole genannt, hergestellt nach bekannten Verfahren, insbesondere der Polykondensation von Hydroxycarbonsäuren bzw. Lactonen oder der Polykondensation von aliphatischen und/oder aromatischen Polycarbonsäuren mit zwei- oder mehrwertigen Alkoholen. Bevorzugt sind Polyesterdiole aus der Umsetzung von zweiwertigen Alkoholen wie insbesondere 1,2-Ethandiol, Diethylenglykol, 1,2-Propandiol, Dipropylenglykol, 1,4-Butandiol, 1,5-Pentandiol, 1,6-Hexandiol, Neopentylglykol, Glycerin, 1,1,1-Trimethylolpropan oder Mischungen der vorgenannten Alkohole, mit organischen Dicarbonsäuren oder deren Anhydride oder Ester, wie insbesondere Bernsteinsäure, Glutarsäure, Adipinsäure, Korksäure, Sebacinsäure, Dodecandicarbonsäure, Maleinsäure, Fumarsäure, Phthalsäure, Isophthalsäure, Terephthalsäure oder Hexahydrophthalsäure oder Mischungen der vorgenannten Säuren, oder Polyesterpolyole aus Lactonen wie insbesondere ε-Caprolacton. Besonders bevorzugt sind Polyesterpolyole aus Adipinsäure oder Sebacinsäure oder Dodecandicarbonsäure und Hexandiol oder Neopentylglykol.
- Polycarbonatpolyole, wie sie durch Umsetzung beispielsweise der oben genannten - zum Aufbau der Polyesterpolyole eingesetzten - Alkohole mit Dialkylcarbonaten, Diarylcarbonaten oder Phosgen zugänglich sind.
- Mindestens zwei Hydroxylgruppen tragende Blockcopolymere, welche mindestens zwei verschiedene Blöcke mit Polyether-, Polyester- und/oder Polycarbonatstruktur der oben beschriebenen Art aufweisen, insbesondere Polyetherpolyesterpolyole.
- Polyacrylat- und Polymethacrylatpolyole.
- Polyhydroxyfunktionelle Fette und Öle, beispielsweise natürliche Fette und Öle, inbesondere Ricinusöl; oder durch chemische Modifizierung von natürlichen Fetten und Ölen gewonnene - sogenannte oleochemische - Polyole, beispielsweise die durch Epoxidierung ungesättigter Öle und anschliessender Ringöffnung mit Carbonsäuren bzw. Alkoholen erhaltenen Epoxypolyester bzw. Epoxypolyether, oder durch Hydroformylierung und Hydrierung ungesättigter Öle erhaltene Polyole; oder aus natürlichen Fetten und Ölen durch Abbauprozesse wie Alkoholyse oder Ozonolyse und anschliessender chemischer Verknüpfung, beispielsweise durch Umesterung oder Dimerisierung, der so gewonnenen Abbauprodukte oder Derivaten davon erhaltene Polyole. Geeignete Abbauprodukte von natürlichen Fetten und Ölen sind insbesondere Fettsäuren und Fettalkohole sowie Fettsäureester, insbesondere die Methylester (FAME), welche beispielsweise durch Hydroformylierung und Hydrierung zu Hydroxyfettsäureestern derivatisiert werden können.
- Polykohlenwasserstoffpolyole, auch Oligohydrocarbonole genannt, wie beispielsweise polyhydroxyfunktionelle Polyolefine, Polyisobutylene, Polyisoprene; polyhydroxyfunktionelle Ethylen-Propylen-, Ethylen-Butylen- oder Ethylen-Propylen-Dien-Copolymere, wie sie beispielsweise von der Firma Kraton Polymers hergestellt werden; polyhydroxyfunktionelle Polymere von Dienen, insbesondere von 1,3-Butadien, welche insbesondere auch aus anionischer Polymerisation hergestellt sein können; polyhydroxyfunktionelle Copolymere aus Dienen wie 1,3-Butadien oder Diengemischen und Vinylmonomeren wie Styrol, Acrylonitril, Vinylchlorid, Vinylacetat, Vinylalkohol, Isobutylen und Isopren, beispielsweise polyhydroxyfunktionelle Acrylonitril/ Butadien-Copolymere, wie sie beispielsweise aus Epoxiden oder Aminoalkoholen und carboxylterminierten Acrylonitril/Butadien-Copolymeren (beispielsweise kommerziell erhältlich unter dem Namen Hypro^{®} CTBN oder CTBNX oder ETBN von Emerald Performance Materials) hergestellt werden können; sowie hydrierte polyhydroxyfunktionelle Polymere oder Copolymere von Dienen.

Geeignet sind insbesondere auch Mischungen von Polyolen.

Bevorzugt sind Polyetherpolyole, Polyesterpolyole, Polycarbonatpolyole, Poly-(meth)acrylatpolyole oder Polybutadienpolyole.

Besonders bevorzugt sind Polyetherpolyole, Polyesterpolyole, insbesondere aliphatische Polyesterpolyole, oder Polycarbonatpolyole, insbesondere aliphatische Polycarbonatpolyole.

Am meisten bevorzugt sind Polyetherpolyole, insbesondere Polyoxypropylendi- oder -triole oder Ethylenoxid-terminierte Polyoxypropylendi- oder -triole. Bevorzugt sind Polyole mit einem mittleren Molekulargewicht im Bereich von 400 bis 20'000 g/mol, bevorzugt von 1'000 bis 15'000 g/mol.

Bevorzugt sind Polyole mit einer mittleren OH-Funktionalität im Bereich von 1.6 bis 3.

Bevorzugt sind bei Raumtemperatur flüssige Polyole.

Bei der Herstellung eines Isocyanatgruppen-haltigen Polyurethanpolymers können auch Anteile von zwei- oder mehrfunktionellen Alkoholen mitverwendet werden, insbesondere 1,2-Ethandiol, 1,2-Propandiol, 1,3-Propandiol, 2-Methyl-1,3-propandiol, 1,2-Butandiol, 1,3-Butandiol, 1,4-Butandiol, 1,3-Pentandiol, 1,5-Pentandiol, 3-Methyl-1,5-pentandiol, Neopentylglykol, Dibromneopentylglykol, 1,2-Hexandiol, 1,6-Hexandiol, 1,7-Heptandiol, 1,2-Octandiol, 1,8-Octandiol, 2-Ethyl-1,3-hexandiol, Diethylenglykol, Triethylenglykol, Dipropylenglykol, Tripropylenglykol, 1,3- oder 1,4-Cyclohexandimethanol, ethoxyliertes Bisphenol-A, propoxyliertes Bisphenol-A, Cyclohexandiol, hydriertes Bisphenol-A, Dimerfettsäurealkohole, 1,1,1-Trimethylolethan, 1,1,1-Trimethylolpropan, Glycerin, Pentaerythritol, Zuckeralkohole wie insbesondere Xylit, Sorbit oder Mannit oder Zucker wie insbesondere Saccharose oder alkoxylierte Derivate der genannten Alkohole oder Mischungen der genannten Alkohole.

Das Isocyanatgruppen-haltige Polyurethanpolymer weist bevorzugt ein mittleres Molekulargewicht im Bereich von 1'500 bis 20'000 g/mol, insbesondere 2'000 bis 15'000 g/mol, auf.

Bevorzugt ist es bei Raumtemperatur flüssig.

Für die Verwendung in einem Heissschmelzklebstoff ist ein bei Raumtemperatur festes Polyurethanpolymer bevorzugt, welches ausgehend von mindestens einem bei Raumtemperatur festen Polyol hergestellt ist. Ein geeignetes bei Raumtemperatur festes Polyol ist bei Raumtemperatur kristallin, teilkristallin oder amorph. Bevorzugt liegt sein Schmelzpunkt im Bereich von 50 bis 180°C, insbesondere 70 bis 150°C. Bevorzugt sind Polyesterpolyole, insbesondere solche abgeleitet von Hexandiol und Adipinsäure oder Dodecandicarbonsäure, oder Acrylatpolyole. Das Polyurethanpolymer wird insbesondere bei einer Temperatur oberhalb des Schmelzpunktes des bei Raumtemperatur festen Polymers hergestellt.

Bevorzugt enthält die Zusammensetzung mindestens ein Isocyanatgruppen-haltiges Polyurethanpolymer.

Zusätzlich zu einem Isocyanatgruppen-haltigen Polyurethanpolymer kann die Zusammensetzung weiterhin mindestens ein Diisocyanat und/oder ein Oligomer oder Polymer eines Diisocyanates enthalten, insbesondere ein IPDI-Isocyanurat oder ein TDI-Oligomer oder ein gemischtes Isocyanurat auf Basis TDI/HDI oder ein HDI-Oligomer oder eine bei Raumtemperatur flüssige Form von MDI.

Eine bei Raumtemperatur flüssige Form von MDI stellt entweder durch partielle chemische Modifizierung - insbesondere Carbodiimidisierung bzw. Uretonimin¬bildung oder Adduktbildung mit Polyolen - verflüssigtes 4,4'-MDI dar, oder es ist ein durch Abmischen gezielt herbeigeführtes oder durch den Herstellungs¬prozess bedingtes Gemisch von 4,4'-MDI mit anderen MDI-Isomeren (2,4'-MDI und/oder 2,2'-MDI), und/oder MDI-Oligomeren und/oder MDI-Homologen (PMDI).

Bevorzugt enthält die Zusammensetzung neben mindestens einem Polyisocyanat und/oder Isocyanatgruppen-haltigen Polyurethanpolymer und mindestens einem Polyaldimin der Formel (IV) und zusätzlich einen oder mehrere weitere Bestandteile, welche insbesondere ausgewählt sind aus Katalysatoren, Füllstoffen, Weichmachern und Lösemitteln.

Geeignete Katalysatoren sind insbesondere Katalysatoren für die Hydrolyse von Aldiminogruppen, insbesondere organische Säuren, insbesondere Carbonsäuren wie 2-Ethylhexansäure, Laurinsäure, Stearinsäure, Isostearinsäure, Ölsäure, Neodecansäure, Benzoesäure, Salicylsäure oder 2-Nitrobenzoesäure, organische Carbonsäureanhydride wie Phthalsäureanhydrid, Hexahydrophthalsäureanhydrid oder Hexahydromethylphthalsäureanhydrid, Silylester von Carbonsäuren, organische Sulfonsäuren wie Methansulfonsäure, p-Toluolsulfonsäure oder 4-Dodecylbenzolsulfonsäure, Sulfonsäureester, andere organische oder anorganische Säuren, oder Mischungen der vorgenannten Säuren und Säureester. Besonders bevorzugt sind Carbonsäuren, insbesondere aromatische Carbonsäuren wie Benzoesäure, 2-Nitrobenzoesäure oder insbesondere Salicylsäure.

Geeignete Katalysatoren sind weiterhin Katalysatoren für die Beschleunigung der Reaktion von Isocyanatgruppen, insbesondere Organozinn(IV)-Verbindungen wie insbesondere Dibutylzinndiacetat, Dibutylzinndilaurat, Dibutylzinndichlorid, Dibutylzinndiacetylacetonat, Dimethylzinndilaurat, Dioctylzinndiacetat, Dioctylzinndilaurat oder Dioctylzinndiacetylacetonat, Komplexverbindungen von Bismut(III) oder Zirkonium(IV), insbesondere mit Liganden ausgewählt aus Alkoholaten, Carboxylaten, 1,3-Diketonaten, Oxinat, 1,3-Ketoesteraten und 1,3-Ketoamidaten, oder tertiäre Aminogruppen enthaltende Verbindungen wie insbesondere 2,2'-Dimorpholinodiethylether (DMDEE).

Geeignet sind insbesondere auch Kombinationen von verschiedenen Katalysatoren.

Geeignete Füllstoffe sind insbesondere gemahlene oder gefällte Calciumcarbonate, welche gegebenenfalls mit Fettsäuren, insbesondere Stearaten, beschichtet sind, Baryte (Schwerspate), Quarzmehle, Quarzsande, Dolomite, Wollastonite, Kaoline, calcinierte Kaoline, Schichtsilikate wie Glimmer oder Talk, Zeolithe, Aluminiumhydroxide, Magnesiumhydroxide, Kieselsäuren inklusive hochdisperse Kieselsäuren aus Pyrolyseprozessen, Zemente, Gipse, Flugaschen, industriell hergestellte Russe, Graphit, Metall-Pulver, beispielsweise von Aluminium, Kupfer, Eisen, Silber oder Stahl, PVC-Pulver oder Hohlkugeln.

Geeignete Weichmacher sind insbesondere Carbonsäureester wie Phthalate, insbesondere Diisononylphthalat (DINP), Diisodecylphthalat (DIDP) oder Di(2-propylheptyl)phthalat (DPHP), hydrierte Phthalate, insbesondere hydriertes Diisononylphthalat bzw. Diisononylcyclohexan-1,2-dicarboxylat (DINCH), Terephthalate, insbesondere Dioctylterephthalat, Trimellitate, Adipate, insbesondere Dioctyladipat, Azelate, Sebacate, Benzoate, Glykolether, Glykolester, organische Phosphor- oder Sulfonsäureester, Polybutene, Polyisobutene oder von natürlichen Fetten oder Ölen abgeleitete Weichmacher, insbesondere epoxidiertes Soja- oder Leinöl.

Geeignete Lösemittel sind insbesondere Aceton, Methylethylketon, Methyl-n-propylketon, Diisobutylketon, Methylisobutylketon, Methyl-n-amylketon, Methylisoamylketon, Acetylaceton, Mesityloxid, Cyclohexanon, Methylcyclohexanon, Ethylacetat, Propylacetat, Butylacetat, n-Butylpropionat, Diethylmalonat, 1-Methoxy-2-propylacetat, Ethyl-3-ethoxypropionat, Diisopropylether, Diethylether, Dibutylether, Diethylenglykoldiethylether, Ethylenglykoldiethylether, Ethylenglykolmonopropylether, Ethylenglykolmono-2-ethylhexylether, Toluol, Xylol, Heptan, Octan, Naphtha, White Spirit, Petrolether oder Benzin, insbesondere Solvesso^{™}-Typen (von Exxon), weiterhin Methylenchlorid, Propylencarbonat, Butyrolacton, N-Methylpyrrolidon oder N-Ethylpyrrolidon.

Die Zusammensetzung kann weitere für Polyurethanzusammensetzungen gebräuchliche Zusätze enthalten. Insbesondere können die folgenden Hilfs- und Zusatzstoffe vorhanden sein:
- anorganische oder organische Pigmente, insbesondere Titandioxid, Chromoxide oder Eisenoxide;
- Fasern, insbesondere Glasfasern, Kohlefasern, Metallfasern, Keramikfasern, Kunststofffasern wie Polyamidfasern oder Polyethylenfasern, oder Naturfasern wie Wolle, Cellulose, Hanf oder Sisal;
- Farbstoffe;
- Trocknungsmittel, insbesondere Molekularsiebpulver, Calciumoxid, hochreaktive Isocyanate wie p-Tosylisocyanat, monomere Diisocyanate oder Orthoameisensäureester;
- Haftvermittler, insbesondere Organoalkoxysilane, insbesondere Epoxysilane wie insbesondere 3-Glycidoxypropyltrimethoxysilan oder 3-Glycidoxypropyltriethoxysilan, (Meth)acrylosilane, Anhydridosilane, Carbamatosilane, Alkylsilane oder Iminosilane, oder oligomere Formen dieser Silane, oder Titanate;
- weitere latente Härter oder Vernetzer, insbesondere Aldimine, Ketimine, Enamine oder Oxazolidine;
- weitere Katalysatoren, welche die Reaktion der Isocyanatgruppen beschleunigen, insbesondere Salze, Seifen oder Komplexe von Zinn, Zink, Bismut, Eisen, Aluminium, Molybdän, Dioxomolybdän, Titan, Zirkonium oder Kalium, insbesondere Zinn(II)-2-ethylhexanoat, Zinn(II)-neodecanoat, Zink(II)-acetat, Zink(II)-2-ethylhexanoat, Zink(II)-laurat, Zink(II)-acetylacetonat, Aluminiumlactat, Aluminiumoleat, Diisopropoxytitan-bis-(ethylacetoacetat) oder Kaliumacetat; tertiäre Aminogruppen enthaltende Verbindungen, insbesondere N-Ethyldiisopropylamin, N,N,N',N'-Tetramethylalkylendiamine, Pentamethylalkylentriamine und höhere Homologe davon, Bis-(N,N-diethylaminoethyl)-adipat, Tris(3-dimethylaminopropyl)amin, 1,4-Diazabicyclo[2.2.2]octan (DABCO), 1,8-Diazabicyclo[5.4.0]undec-7-en (DBU), 1,5-Diazabicyclo[4.3.0]-non-5-en (DBN), N-Alkylmorpholine, N,N'-Dimethylpiperazin; stickstoffaromatische Verbindungen wie 4-Dimethylamino-pyridin, N-Methylimidazol, N-Vinylimidazol oder 1,2-Dimethylimidazol; organische Ammoniumverbindungen wie Benzyltrimethylammoniumhydroxid oder alkoxylierte tertiäre Amine; sogenannte "delayed action"-Katalysatoren, welche Modifizierungen bekannter Metall- oder Aminkatalysatoren darstellen;
- Rheologie-Modifizierer, insbesondere Verdickungsmittel, insbesondere Schichtsilikate wie Bentonite, Derivate von Rizinusöl, hydriertes Rizinusöl, Polyamide, Polyamidwachse, Polyurethane, Harnstoffverbindungen, pyrogene Kieselsäuren, Celluloseether oder hydrophob modifizierte Polyoxyethylene;
- natürliche Harze, Fette oder Öle wie Kolophonium, Schellack, Leinöl, Rizinusöl oder Sojaöl;
- nicht-reaktive Polymere, insbesondere Homo- oder Copolymere von ungesättigten Monomeren, insbesondere aus der Gruppe umfassend Ethylen, Propylen, Butylen, Isobutylen, Isopren, Vinylacetat oder Alkyl(meth)acrylate, insbesondere Polyethylene (PE), Polypropylene (PP), Polyisobutylene, Ethylenvinylacetat-Copolymere (EVA) oder ataktische Poly-α-Olefine (APAO);
- flammhemmende Substanzen, insbesondere die bereits genannten Füllstoffe Aluminiumhydroxid oder Magnesiumhydroxid, sowie insbesondere organische Phosphorsäureester wie insbesondere Triethylphosphat, Trikresylphosphat, Triphenylphosphat, Diphenylkresylphosphat, Isodecyldiphenylphosphat, Tris(1,3-dichlor-2-propyl)phosphat, Tris(2-chlorethyl)phosphat, Tris(2-ethylhexyl)phosphat, Tris(chlorisopropyl)phosphat, Tris(chlorpropyl)-phosphat, isopropyliertes Triphenylphosphat, Mono-, Bis- oder Tris(isopropylphenyl)phosphate unterschiedlichen Isopropylierungsgrades, Resorcinol-bis(diphenylphosphat), Bisphenol-A-bis(diphenylphosphat) oder Ammoniumpolyphosphate;
- Additive, insbesondere Netzmittel, Verlaufmittel, Entschäumer, Entlüfter, Stabilisatoren gegen Oxidation, Wärme, Licht oder UV-Strahlung oder Biozide;
oder weitere üblicherweise in feuchtigkeitshärtenden Zusammensetzungen eingesetzte Substanzen.

Es kann sinnvoll sein, gewisse Substanzen vor dem Einmischen in die Zusammensetzung chemisch oder physikalisch zu trocknen.

Als weitere latente Härter geeignet sind insbesondere Polyaldimine abgeleitet von 2,2-Dimethyl-3-lauroyloxypropanal.

In der Zusammensetzung liegt das Verhältnis zwischen Aldiminogruppen und Isocyanatgruppen bevorzugt im Bereich von 0.05 bis 1.1, besonders bevorzugt 0.1 bis 1.0, insbesondere 0.2 bis 0.9.

Die Zusammensetzung enthält bevorzugt einen Gehalt an Polyisocyanaten und Isocyanatgruppen-haltigen Polyurethanpolymeren im Bereich von 5 bis 90 Gewichts-%, insbesondere 10 bis 80 Gewichts-%.

Die Zusammensetzung wird insbesondere unter Ausschluss von Feuchtigkeit hergestellt und bei Umgebungstemperatur in feuchtigkeitsdichten Gebinden aufbewahrt. Ein geeignetes feuchtigkeitsdichtes Gebinde besteht insbesondere aus einem gegebenenfalls beschichteten Metall und/oder Kunststoff und stellt insbesondere ein Fass, ein Container, ein Hobbock, ein Eimer, ein Kanister, eine Büchse, ein Beutel, ein Schlauchbeutel, eine Kartusche oder eine Tube dar.

Die Zusammensetzung kann in Form einer einkomponentigen oder in Form einer mehrkomponentigen, insbesondere zweikomponentigen, Zusammensetzung vorliegen.

Als "einkomponentig" wird eine Zusammensetzung bezeichnet, bei welcher alle Bestandteile der Zusammensetzung im gleichen Gebinde vorliegen und welche als solche lagerstabil ist.

Als "zweikomponentig" wird eine Zusammensetzung bezeichnet, bei welcher die Bestandteile der Zusammensetzung in zwei verschiedenen Komponenten vorliegen, welche in voneinander getrennten Gebinden gelagert und erst kurz vor oder während der Applikation der Zusammensetzung miteinander vermischt werden.

Die Zusammensetzung ist bevorzugt einkomponentig. Sie ist bei geeigneter Verpackung und Aufbewahrung lagerstabil, typischerweise während mehreren Monaten bis zu einem Jahr oder länger.

Bei der Applikation der Zusammensetzung beginnt der Prozess der Aushärtung. Als Ergebnis davon entsteht die ausgehärtete Zusammensetzung.

Im Fall einer einkomponentigen Zusammensetzung wird diese als solche appliziert und beginnt darauf unter dem Einfluss von Feuchtigkeit bzw. Wasser auszuhärten. Zur Beschleunigung der Aushärtung kann der Zusammensetzung bei der Applikation eine Beschleuniger-Komponente, welche Wasser und/oder einen Katalysator enthält oder freisetzt, zugemischt werden, oder die Zusammensetzung kann nach ihrer Applikation mit einer solchen Beschleuniger-Komponente in Kontakt gebracht werden.

Im Fall einer zweikomponentigen Zusammensetzung wird diese nach dem Vermischen der beiden Komponenten appliziert und beginnt dabei durch interne Reaktion auszuhärten, wobei die Aushärtung gegebenenfalls durch die Einwirkung von externer Feuchtigkeit vervollständigt wird. Das Vermischen der beiden Komponenten kann kontinuiertlich oder batchweise mit dynamischen Mischern oder Statikmischern erfolgen.

Bei der Aushärtung reagieren die Isocyanatgruppen unter dem Einfluss von Feuchtigkeit mit den Aldiminogruppen des Polyaldimins der Formel (IV) und gegebenenfalls vorhandenen weiteren blockierten Aminogruppen. Ein Teil der Isocyanatgruppen, insbesondere die gegenüber den Aldiminogruppen überschüssigen, reagieren unter dem Einfluss von Feuchtigkeit untereinander und/oder mit gegebenenfalls in der Zusammensetzung vorhandenen weiteren Reaktivgruppen, insbesondere Hydroxylgruppen oder freien Aminogruppen. Die Gesamtheit dieser zur Aushärtung der Zusammensetzung führenden Reaktionen der Isocyanatgruppen wird auch als Vernetzung bezeichnet.

Die zur Aushärtung der einkomponentigen Zusammensetzung benötigte Feuchtigkeit gelangt bevorzugt aus der Luft (Luftfeuchtigkeit) durch Diffusion in die Zusammensetzung. Dabei bildet sich an den mit Luft in Kontakt stehenden Oberflächen der Zusammensetzung eine feste Schicht ausgehärteter Zusammensetzung ("Haut"). Die Aushärtung setzt sich entlang der Diffusionsrichtung von aussen nach innen fort, wobei die Haut zunehmend dicker wird und schliesslich die ganze applizierte Zusammensetzung umfasst. Die Feuchtigkeit kann zusätzlich oder vollständig auch aus einem oder mehreren Substrat(en), auf welche(s) die Zusammensetzung appliziert wurde, in die Zusammensetzung gelangen und/oder aus einer Beschleuniger-Komponente stammen, welche der Zusammensetzung bei der Applikation zugemischt oder nach der Applikation mit dieser in Kontakt gebracht wird, beispielsweise durch Bestreichen oder Besprühen.

Die zur Vervollständigung der Aushärtung einer zweikomponentigen Zusammensetzung gegebenenfalls benötigte externe Feuchtigkeit stammt bevorzugt aus der Luft und/oder aus den Substraten.

Die Zusammensetzung wird bevorzugt bei Umgebungstemperatur appliziert, insbesondere im Bereich von etwa 0 bis 50°C, bevorzugt im Bereich von 5 bis 40°C.

Falls die Zusammensetzung einen Heissschmelzklebstoff darstellt, wird sie bevorzugt im geschmolzenen Zustand bei einer Temperatur im Bereich von 80 bis 180°C appliziert.

Die Aushärtung der Zusammensetzung erfolgt bevorzugt bei Umgebungstemperatur.

Die Zusammensetzung verfügt über eine vergleichsweise lange Offenzeit.

Als "Offenzeit" wird die Zeitspanne bezeichnet, während der die Zusammensetzung verarbeitet oder nachbearbeitet werden kann, nachdem der Aushärtungsprozess begonnen hat.

Die Zeit bis zur Ausbildung einer Haut ("Hautbildungszeit") bzw. bis zur Klebefreiheit ("tack-free time") stellt dabei ein Mass für die Offenzeit dar.

Bei der Vernetzung wird das Blockierungsmittel, d.h. das beschriebene Aldehydgemisch, freigesetzt. Dieses ist weitgehend unflüchtig und geruchlos und verbleibt zum grössten Teil in der ausgehärteten Zusammensetzung. Es verhält sich bzw. wirkt wie ein Weichmacher. Als solcher kann es grundsätzlich selber migrieren und/oder die Migration von Weichmachern beeinflussen. Das Blockierungsmittel ist mit der ausgehärteten Zusammensetzung sehr gut verträglich, migriert selber kaum und löst auch keine verstärkte Migration von weiteren Weichmachern aus.

Bevorzugt stellt die Zusammensetzung einen Klebstoff oder einen Dichtstoff oder eine Beschichtung dar.

Bevorzugt ist der Klebstoff oder Dichtstoff oder die Beschichtung elastisch.

Als Klebstoff und/oder Dichtstoff ist die Zusammensetzung insbesondere geeignet für Klebe- und Abdichtungsanwendungen, insbesondere in der Bau- und Fertigungsindustrie oder im Fahrzeugbau, insbesondere für die Parkettverklebung, Anbauteilverklebung, Hohlraumversiegelung, Montage, Modulverklebung, Karosserieverklebung, Scheibenverklebung oder Fugenabdichtung. Elastische Verklebungen im Fahrzeugbau sind beispielsweise das Ankleben von Teilen wie Kunststoffabdeckungen, Zierleisten, Flansche, Stosstangen, Führerkabinen oder andere Anbauteile, an die lackierte Karosserie eines Fahrzeugs, oder das Einkleben von Scheiben in die Karosserie, wobei die Fahrzeuge insbesondere Automobile, Lastkraftwagen, Busse, Schienenfahrzeuge oder Schiffe darstellen.

Als Dichtstoff ist die Zusammensetzung insbesondere geeignet für das elastische Abdichten von Fugen, Nähten oder Hohlräumen aller Art, insbesondere von Fugen am Bau wie Dilatationsfugen oder Anschlussfugen zwischen Bauteilen. Insbesondere für das Abdichten von Dilatationsfugen an Bauwerken ist ein Dichtstoff mit weichelastischen Eigenschaften besonders geeignet.

Als Beschichtung ist die Zusammensetzung geeignet zum Schutz von Böden oder Mauern, insbesondere als Beschichtung von Balkonen, Terrassen, Plätzen, Brücken, Parkdecks, oder zur Abdichtung von Dächern, insbesondere Flachdächern oder schwach geneigten Dachflächen oder Dachgärten, oder im Innern von Gebäuden zur Wasserabdichtung, beispielsweise unter Fliesen oder Keramikplatten in Nasszellen oder Küchen, oder als Bodenbelag in Küchen, Industriehallen oder Fabrikationsräumen, oder als Abdichtung in Auffangwannen, Kanälen, Schächten oder Abwasserbehandlungsanlagen, oder zum Schutz von Oberflächen als Lack oder Versiegelung, oder als Vergussmasse zur Hohlraumversiegelung, als Nahtabdichtung oder als Schutzbeschichtung für beispielsweise Rohre.

Sie kann auch zu Reparaturzwecken als Abdichtung oder Beschichtung verwendet werden, beispielsweise von undichten Dachmembranen oder nicht mehr tauglichen Bodenbelägen oder insbesondere als Reparaturmasse für hochreaktive Spritzabdichtungen.

Die Zusammensetzung kann so formuliert sein, dass sie eine pastöse Konsistenz mit strukturviskosen Eigenschaften aufweist. Eine solche Zusammensetzung wird mittels einer geeigneten Vorrichtung appliziert, beispielsweise aus handelsüblichen Kartuschen oder Fässern oder Hobbocks, beispielsweise in Form einer Raupe, wobei diese eine im Wesentlichen runde oder dreieckige Querschnittsfläche aufweisen kann.

Die Zusammensetzung kann weiterhin so formuliert sein, dass sie flüssig und sogenannt selbstverlaufend oder nur leicht thixotrop ist und zur Applikation ausgegossen werden kann. Als Beschichtung kann sie beispielsweise anschliessend flächig bis zur erwünschten Schichtdicke verteilt werden, beispielsweise mittels einer Rolle, einem Schieber, einer Zahntraufel oder einer Spachtel. Dabei wird in einem Arbeitsgang typischerweise eine Schichtdicke im Bereich von 0.5 bis 3 mm, insbesondere 1.0 bis 2.5 mm, aufgetragen.

Geeignete Substrate, welche mit der Zusammensetzung verklebt oder abgedichtet oder beschichtet werden können, sind insbesondere
- Glas, Glaskeramik, Beton, Mörtel, Faserzement, insbesondere Faserzement-Platten, Backstein, Ziegel, Gips, insbesondere Gips-Platten, oder Natursteine wie Granit oder Marmor;
- Reparatur- oder Nivelliermassen auf Basis PCC (Polymer-modifizierter Zementmörtel) oder ECC (Epoxidharz-modifizierter Zementmörtel);
- Metalle oder Legierungen wie Aluminium, Kupfer, Eisen, Stahl, Buntmetalle, inklusive oberflächenveredelte Metalle oder Legierungen wie verzinkte oder verchromte Metalle;
- Asphalt oder Bitumen;
- Leder, Textilien, Papier, Holz, mit Harzen wie Phenol-, Melamin- oder Epoxidharzen gebundene Holzwerkstoffe, Harz-Textil-Verbundwerkstoffe oder weitere sogenannte Polymer-Composites;
- Kunststoffe wie Hart- und Weich-PVC, Polycarbonat, Polystyrol, Polyester, Polyamid, PMMA, ABS, SAN, Epoxidharze, Phenolharze, PUR, POM, TPO, PE, PP, EPM oder EPDM, jeweils unbehandelt oder oberflächenbehandelt, beispielsweise mittels Plasma, Corona oder Flammen;
- Faserverstärkte Kunststoffe, wie Kohlefaser-verstärkte Kunststoffe (CFK), Glasfaser-verstärkte Kunststoffe (GFK) und Sheet Moulding Compounds (SMC);
- Isolierschäume, insbesondere aus EPS, XPS, PUR, PIR, Steinwolle, Glaswolle oder geschäumtem Glas (Foamglas);
- beschichtete oder lackierte Substrate, insbesondere lackierte Fliesen, gestrichener Beton, pulverbeschichtete Metalle oder Legierungen oder lackierte Bleche;
- Farben oder Lacke, insbesondere Automobildecklacke.

Die Substrate können bei Bedarf vor dem Applizieren vorbehandelt werden, insbesondere durch physikalische und/oder chemische Reinigungsverfahren oder das Aufbringen eines Aktivators oder eines Primers.

Verklebt und/oder abgedichtet werden können zwei gleichartige oder zwei verschiedene Substrate.

Aus der Applikation und Aushärtung der Zusammensetzung wird ein Artikel erhalten, welcher mit der Zusammensetzung verklebt oder abgedichtet oder beschichtet ist. Dieser Artikel kann ein Bauwerk oder ein Teil davon sein, insbesondere ein Bauwerk des Hoch- oder Tiefbaus, eine Brücke, ein Dach, ein Treppenhaus oder eine Fassade, oder er kann ein industrielles Gut oder ein Konsumgut sein, insbesondere ein Fenster, ein Rohr, ein Rotorblatt einer Windkraftanlage, eine Haushaltmaschine oder ein Transportmittel wie insbesondere ein Automobil, ein Bus, ein Lastkraftwagen, ein Schienenfahrzeug, ein Schiff, ein Flugzeug oder ein Helikopter, oder ein Anbauteil davon.

Die erfindungsgemässe Zusammensetzung weist vorteilhafte Eigenschaften auf. Sie ist unter Ausschluss von Feuchtigkeit lagerstabil, auch zusammen mit den sehr reaktiven aromatischen Isocyanatgruppen. Sie verfügt über eine ausreichend lange Offenzeit, um über einen gewissen Zeitraum nach der Applikation einen nahtlosen Verlauf des applizierten Materials oder eine Positionierung oder Nachjustierung der damit verbundenen Objekte zu ermöglichen, was beispielsweise bei grossflächigen Beschichtungen, langen Dichtstreifen oder bei der Verklebung von grossen oder komplexen Objekten wichtig ist. Die Aushärtung erfolgt ohne störende Geruchsimmissionen schnell, zuverlässig und blasenfrei, so dass die Zusammensetzung ohne Einschränkung auch unter klimatisch ungünstigen Bedingungen wie hoher Luftfeuchtigkeit und/oder hoher Temperatur oder unter Einsatz von wasserhaltigen Beschleunigerkomponenten verwendbar ist sowie bedenkenlos auch in Innenräumen oder bei grossflächigen Anwendungen eingesetzt werden kann. Bei der Aushärtung baut sie rasch Festigkeit auf, wobei die gebildete Haut auf der Oberfläche schon bald überraschend nicht-klebrig trocken ist, was insbesondere bei der Anwendung auf Baustellen sehr wertvoll ist, da dadurch Verschmutzungen durch beispielsweise Staub verhindert werden. Die Aushärtung verläuft vollständig und führt zu einem hochwertigen Material, welches eine hohe mechanische Festigkeit und Dehnbarkeit mit einem moderaten Elastizitätsmodul verbindet und sich somit auch für weichelastische Produkte eignet. Trotz ihres Gehalts an freigesetztem unflüchtigem Blockierungsmittel zeigt die Zusammensetzung kaum durch Weichmachermigration ausgelöste Fehler wie Ausschwitzen ("Bleeding"), Verfärbung, Fleckenbildung, Erweichung oder Substratablösung und kann somit ohne Einschränkung auch auf porösen Substraten oder Spannungsriss-bildenden Kunststoffen und in Kombination mit Deckschichten eingesetzt werden.

### Beispiele

Im Folgenden sind Ausführungsbeispiele aufgeführt, welche die beschriebene Erfindung näher erläutern sollen. Selbstverständlich ist die Erfindung nicht auf diese beschriebenen Ausführungsbeispiele beschränkt.

Als "Normklima" ("NK") wird eine Temperatur von 23±1°C und eine relative Luftfeuchtigkeit von 50±5% bezeichnet.

### Beschreibung der Messmethoden:

Die **Aminzahl** (inklusive blockierte Aminogruppen) wurde bestimmt mittels Titration (mit 0.1N HClO₄ in Essigsäure gegen Kristallviolett).

Die **Viskosität** wurde mit einem thermostatisierten Kegel-Platten-Viskosimeter Rheotec RC30 (Kegeldurchmesser 50 mm, Kegelwinkel 1°, Kegelspitze-Platten-Abstand 0.05 mm, Scherrate 10 s⁻¹) gemessen.

Die **Kristallisationstemperatur** bzw. Glasübergangstemperatur wurde mittels DSC mit einem Mettler Toledo DSC 3+ 700 Gerät in einem Temperaturbereich von -80 bis +50°C mit einer Aufheizrate von 5 K/min bestimmt.

### Verwendete Aldehyde:

Aldehyd-1: Rohprodukt aus mittels HF-BF₃ katalysierter Formylierung von C₁₀₋₁₄-Alkylbenzol mit Kohlenmonoxid nach destillativer Entfernung der niedermolekularen Bestandteile, enthaltend 85-90 Gewichts-% mehrheitlich verzweigte 4-(C₁₀₋₁₄-Alkyl)benzaldehyde und 10-15 Gewichts-% höhersiedende bzw. höhermolekulare Alkylbenzol-Verbindungen (gem. GC-FID); mittleres Aldehyd-Equivalentgewicht 309 g/Eq; Gardner-Farbzahl 10.
Aldehyd-2: Fraktioniertes Aldehydgemisch aus der über Kopf Destillation von Aldehyd-1 im Hochvakuum, enthaltend >98 Gewichts-% mehrheitlich verzweigte 4-(C₁₀₋₁₄-Alkyl)benzaldehyde (gem. GC-FID); mittleres Aldehyd-Equivalentgewicht 290 g/Eq; Gardner-Farbzahl 3.
Aldehyd-3: 2,2-Dimethyl-3-lauroyloxypropanal (284.4 g/mol)

Beim Aldehyd-1 handelt es sich um ein erfindungsgemässes Aldehydgemisch. Der entsprechende destillativ aufgereinigte Aldehyd-2, bei welchem die höhersiedenden Nebenprodukte abgetrennt waren, sowie der Aldehyd-3 dienen als Vergleich.

### Herstellung von blockierten Aminen bzw. latenten Härtern:

### Aldimin A-1:

106.06 g Aldehyd-1 wurden in einem Rundkolben unter Stickstoffatmosphäre vorgelegt. Unter Rühren wurden 27.86 g 3-Aminomethyl-3,5,5-trimethylcyclohexylamin (Vestamin^{®} IPD, von Evonik) zugegeben und anschliessend die flüchtigen Bestandteile bei 80°C und 10 mbar Vakuum entfernt. Es wurde eine hellgelbe, geruchlose Flüssigkeit mit einer Viskosität von 10.7 Pa s bei 20°C, einer Aminzahl von 143.5 mg KOH/g und einer Kristallisationstemperatur von - 46.1°C erhalten.

### Aldimin A-2:

106.06 g Aldehyd-1 wurden in einem Rundkolben unter Stickstoffatmosphäre vorgelegt. Unter Rühren wurden 27.20 g einer 70%igen wässrigen Lösung von 1,6-Hexandiamin (von Sigma-Aldrich) zugegeben und anschliessend die flüchtigen Bestandteile bei 80°C und 10 mbar Vakuum entfernt. Es wurde eine hellgelbe, geruchlose Flüssigkeit mit einer Viskosität von 0.7 Pa s bei 20°C, einer Aminzahl von 158.6 mg KOH/g und einer Kristallisationstemperatur von - 66.5°C erhalten.

### Aldimin R-1:

100.00 g Aldehyd-2 wurden in einem Rundkolben unter Stickstoffatmosphäre vorgelegt. Unter Rühren wurden 27.86 g 3-Aminomethyl-3,5,5-trimethylcyclohexylamin (Vestamin^{®} IPD, von Evonik) zugegeben und anschliessend die flüchtigen Bestandteile bei 80°C und 10 mbar Vakuum entfernt. Es wurde eine blassgelbe, geruchlose Flüssigkeit mit einer Viskosität von 25.3 Pa s bei 20°C, einer Aminzahl von 152.1 mg KOH/g und einer Kristallisationstemperatur von -40.6°C erhalten.

### Aldimin R-2:

100.00 g Aldehyd-2 wurden in einem Rundkolben unter Stickstoffatmosphäre vorgelegt. Unter Rühren wurden 27.20 g einer 70%igen wässrigen Lösung von 1,6-Hexandiamin (von Sigma-Aldrich) zugegeben und anschliessend die flüchtigen Bestandteile bei 80°C und 10 mbar Vakuum entfernt. Es wurde eine blassgelbe, geruchlose Flüssigkeit mit einer Viskosität von 1.0 Pa s bei 20°C, einer Aminzahl von 169.4 mg KOH/g und einer Kristallisationstemperatur von -63.8°C erhalten.

### Aldimin R-3:

50.00 g Aldehyd-3 wurden in einem Rundkolben unter Stickstoffatmosphäre vorgelegt. Unter Rühren wurden 13.93 g 3-Aminomethyl-3,5,5-trimethylcyclohexylamin (Vestamin^{®} IPD, von Evonik) zugegeben und anschliessend die flüchtigen Bestandteile bei 80°C und 10 mbar Vakuum entfernt. Es wurde eine blassgelbe, geruchlose Flüssigkeit mit einer Viskosität von 0.2 Pa s bei 20°C und einer Aminzahl von 153.0 mg KOH/g erhalten.

Die Aldimine **A-1** und **A-2** sind erfindungsgemässe latente Härter und entsprechen der Formel (IV).

Die Aldimine **R-1** und **R-2** und **R-3** dienen als Vergleich.

### Herstellung von Isocyanatgruppen-haltigen Polymeren

### Polymer P1:

400 g Polyoxypropylen-Diol (Acclaim^{®} 4200, von Covestro; OH-Zahl 28.5 mg KOH/g) und 52 g 4,4'-Diphenylmethandiisocyanat (Desmodur^{®} 44 MC L, von Covestro) wurden nach bekanntem Verfahren bei 80°C zu einem bei Raumtemperatur flüssigen, NCO-terminierten Polyurethanpolymer mit einem Gehalt an freien Isocyanatgruppen von 1.85 Gewichts-% umgesetzt.

### Polymer P2:

590 g Polyoxypropylen-Diol (Acclaim^{®} 4200, von Covestro; OH-Zahl 28.5 mg KOH/g), 1180 g Polyoxypropylenpolyoxyethylen-Triol (Caradol^{®} MD34-02, von Shell; OH-Zahl 35.0 mg KOH/g) und 230 g Isophorondiisocyanat (Vestanat^{®} IPDI, von Evonik) wurden nach bekanntem Verfahren bei 80 °C zu einem bei Raumtemperatur flüssigen, NCO-terminierten Polyurethanpolymer mit einem Gehalt an freien Isocyanatgruppen von 2.10 Gewichts-% umgesetzt.

### Polymer P3:

300.0 g Polyoxypropylenpolyoxyethylen-Diol (Desmophen^{®} L300, von Covestro; OH-Zahl 190.0 mg KOH/g) und 228.8 g Isophorondiisocyanat (Vestanat^{®} IPDI, von Evonik) wurden nach bekanntem Verfahren bei 60°C zu einem bei Raumtemperatur flüssigen, NCO-terminierten Polyurethanpolymer mit einem Gehalt an freien Isocyanatgruppen von 7.91 Gewichts-% umgesetzt.

### Polyurethan-Zusammensetzungen (einkomponentig)

### Zusammensetzungen Z1 bis Z6

Für jede Zusammensetzung wurden die in der Tabelle 1 angegebenen Inhaltsstoffe in den angegebenen Mengen (in Gewichtsteilen) mittels eines Zentrifugalmischers (SpeedMixer^{™} DAC 150, FlackTek Inc.) unter Feuchtigkeitsausschluss während einer Minute bei 3000 rpm vermischt und unter Feuchtigkeitsausschluss aufbewahrt. Jede Zusammensetzung wurde folgendermassen geprüft:
Als Mass für die Lagerstabilität wurden die **Viskosität (1d RT)** am Folgetag der Herstellung und die **Viskosität (7d 60°C)** nach einer Lagerung im verschlossenen Gebinde während 7 Tagen in einem 60 °C warmen Umluftofen bestimmt, wie vorgängig beschrieben.

Als Mass für die Offenzeit wurde die Hautbildungszeit **(tack-free time)** bestimmt. Dazu wurden einige Gramm der Zusammensetzung in einer Schichtdicke von ca. 2 mm auf Pappkarton aufgetragen und im Normklima die Zeitdauer bestimmt, bis beim leichten Antippen der Oberfläche der Zusammensetzung mittels einer Pipette aus LDPE erstmals keine Rückstände auf der Pipette mehr zurückblieben.

Zur Bestimmung der mechanischen Eigenschaften wurde jede Zusammensetzung auf eine PTFE-beschichtete Folie zu einem Film von 2 mm Dicke ausgegossen, während 7 Tagen im Normklima gelagert, einige Hanteln mit einer Länge von 75 mm bei einer Steglänge von 30 mm und einer Stegbreite von 4 mm aus dem Film ausgestanzt und diese gemäss DIN EN 53504 bei einer Zuggeschwindigkeit von 200 mm/min auf **Zugfestigkeit** (Bruchkraft), **Bruchdehnung, E-Modul 5%** (bei 0.5-5% Dehnung) und **E-Modul 50%** (bei 0.5-50% Dehnung) geprüft.

Der **Aspekt** wurde visuell an den hergestellten Filmen beurteilt. Als "schön" wurde ein klarer Film mit nichtklebriger Oberfläche ohne Blasen bezeichnet. Der **Geruch** wurde durch Riechen mit der Nase im Abstand von 2 cm an den frisch hergestellten Filmen beurteilt. "Ja" bedeutet, dass ein Geruch deutlich wahrnehmbar war. "Nein" bedeutet, dass kein Geruch wahrnehmbar war.

Die Resultate sind in der Tabelle 1 angegeben.

Bei den mit (Ref) gekennzeichneten Zusammensetzungen handelt es sich um Vergleichsbeispiele.

**Tabelle 1: Zusammensetzung (in Gewichtsteilen) und Eigenschaften von Z1 bis Z6.**

| **Zusammensetzung** | **Z1** | **Z2 (Ref)** | **Z3** | **Z4 (Ref)** | **Z5** | **Z6 (Ref)** |
|---|---|---|---|---|---|---|
| **Polymer P1** | 80.00 | 80.00 | 80.00 | 80.00 | - | - |
| **Polymer P2** | - | - | - | - | 80.00 | 80.00 |
| **Aldimin** | **A-1** | **R-1** | **A-2** | **R-2** | **A-1** | **R-1** |
| | 9.63 | 9.09 | 8.71 | 8.16 | 10.94 | 10.32 |
| Salicylsäurelösung¹ | 1.50 | 1.50 | 1.50 | 1.50 | 1.50 | 1.50 |
| Viskosität (1d RT) | 29 | 31 | 43 | 51 | 13 | 14 |
| [Pa·s] (7d 60°C) | 31 | 33 | 60 | 80 | 14 | 15 |
| tack-free time | 115' | 105' | 47' | 50' | 195' | 180' |
| Zugfestigkeit [MPa] | 1.50 | 1.44 | 3.25 | 2.40 | 1.27 | 1.23 |
| Bruchdehnung [%] | 1778 | 1809 | 609 | 357 | 237 | 213 |
| E-Modul 5% [MPa] | 0.72 | 0.77 | 6.75 | 6.59 | 1.19 | 1.25 |
| E-Modul 50% | 0.25 | 0.39 | 1.81 | 1.82 | 0.72 | 0.79 |
| Aspekt | schön | schön | schön | schön | schön | schön |
| Geruch | nein | nein | nein | nein | nein | nein |

| | | | | | | |
|---|---|---|---|---|---|---|
| ¹ 5% in Dioctyladipat | | | | | | |

### Zusammensetzungen Z7 bis Z10

Für jede Zusammensetzung wurden die in der Tabelle 2 angegebenen Inhaltsstoffe in den angegebenen Mengen (in Gewichtsteilen) mittels eines Zentrifugalmischers (SpeedMixer^{™} DAC 150, FlackTek Inc.) unter Feuchtigkeitsausschluss während einer Minute bei 3000 rpm vermischt und unter Feuchtigkeitsausschluss aufbewahrt. Jede Zusammensetzung wurde folgendermassen geprüft:
Als Mass für die Weichmachermigration wurde die Fleckenbildung auf Karton bestimmt. Dazu wurde jede Zusammensetzung so auf ein Stück Pappkarton appliziert, dass sie eine runde Grundfläche von 15 mm Durchmesser und eine Höhe von 4 mm aufwies, und während 7 Tagen im NK gelagert. Um jede Zusammensetzung herum war danach auf dem Karton ein dunkler ovaler Fleck entstanden. Dessen Ausmasse (Höhe und Breite) wurden ausgemessen und in der Tabelle 2 als **Migration** angegeben.

**Tabelle 2: Zusammensetzung (in Gewichtsteilen) und Eigenschaften von Z7 bis Z10.**

| **Zusammensetzung** | | **Z7** | **Z8 (Ref)** | **Z9 (Ref)** | **Z10 (Ref)** |
|---|---|---|---|---|---|
| **Polymer P3** | | 15.00 | 15.00 | 15.00 | 15.00 |
| Kreide | | 15.00 | 15.00 | 15.00 | 15.00 |
| Kieselsäure | | 1.13 | 1.13 | 1.13 | 1.13 |
| **Aldimin** | | **A-1** | **R-1** | **R-3** | - |
| | | 5.82 | 5.49 | 5.46 | |
| Salicylsäurelösung¹ | | 3.00 | 3.00 | 3.00 | 3.00 |
| DBTDL-Lösung² | | 1.50 | 1.50 | 1.50 | 1.50 |
| **Migration** | Höhe | 22 | 21 | 33 | 19 |
| **(7d)** [mm] | Breite | 25 | 23 | 28 | 19 |
| **Geruch** | | nein | nein | nein | nein |

| | | | | | |
|---|---|---|---|---|---|
| ¹ 5% in Dioctyladipat ² 5% Dibutylzinndialurat in Diisodecylphthalat | | | | | |

## Patentansprüche

1. Verwendung eines Aldehydgemisches enthaltend
- 70 bis 92 Gewichts-% Aldehyde der Formel (I), wobei R für einen Alkyl-Rest mit 6 bis 20 C-Atomen steht, und 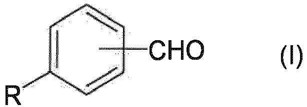
- 8 bis 30 Gewichts-% nicht der Formel (I) entsprechende Alkylbenzol-Verbindungen
als Blockierungsmittel für Amine, **dadurch gekennzeichnet, dass** die nicht der Formel (I) entsprechenden Alkylbenzol-Verbindungen einen höheren Siedepunkt als die Aldehyde der Formel (I) aufweisen.

2. Verwendung gemäss Anspruch 1, **dadurch gekennzeichnet, dass** die Alkyl-Reste R mehrheitlich verzweigt sind.

3. Verwendung gemäss einem der Ansprüche 1 oder 2, **dadurch gekennzeichnet, dass** die Formyl-Gruppe in para-Stellung steht.

4. Verwendung gemäss einem der Ansprüche 1 bis 3, **dadurch gekennzeichnet, dass** die Aldehyde der Formel (I) ausgewählt sind aus 4-Decylbenzaldehyden, 4-Undecylbenzaldehyden, 4-Dodecylbenzaldehyden, 4-Tridecylbenzaldehyden und 4-Tetradecylbenzaldehyden, deren Alkyl-Reste mehrheitlich verzweigt sind.

5. Verwendung gemäss einem der Ansprüche 1 bis 4, **dadurch gekennzeichnet, dass** die nicht der Formel (I) entsprechenden Alkylbenzol-Verbindungen eine oder mehrere Verbindungen der Formeln 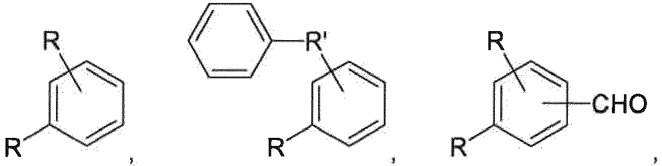 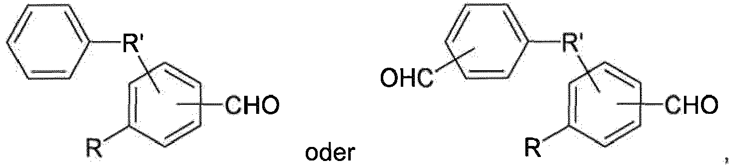 wobei R' für einen Alkylen-Rest mit 6 bis 20 C-Atomen steht, umfassen.

6. Verwendung gemäss einem der Ansprüche 1 bis 5, **dadurch gekennzeichnet, dass** das Aldehydgemisch ein Umsetzungsprodukt einer Formylierung von mindestens einem Alkylbenzol der Formel 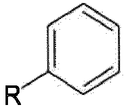 darstellt.

7. Verwendung gemäss Anspruch 6, **dadurch gekennzeichnet, dass** die Formylierung mit Kohlenmonoxid in Gegenwart von Flusssäure und Bortrifluorid durchgeführt wurde.

8. Verwendung gemäss einem der Ansprüche 1 bis 7, **dadurch gekennzeichnet, dass** das zu blockierende Amin mindestens eine primäre oder sekundäre Aminogruppe und zusätzlich mindestens eine Reaktivgruppe ausgewählt aus primärer Aminogruppe, sekundärer Aminogruppe, Hydroxylgruppe und Silangruppe aufweist.

9. Verwendung gemäss einem der Ansprüche 1 bis 8, **dadurch gekennzeichnet, dass** das zu blockierende Amin ausgewählt ist aus der Gruppe bestehend aus primären aliphatischen Diaminen, primären aromatischen Diaminen, primären aliphatischen Triaminen, aliphatischen Diaminen mit einer primären und einer sekundären Aminogruppe, aliphatischen Polyaminen mit zwei primären und einer sekundären Aminogruppe, Aminoalkoholen, Dialkanolaminen, Aminosilanen und Alkanolaminosilanen.

10. Verwendung gemäss einem der Ansprüche 1 bis 9, **dadurch gekennzeichnet, dass** das Aldehydgemisch mit dem zu blockierenden Amin so umgesetzt wird, dass
- das Aldehydgemisch mit dem Amin zu einer Reaktionsmischung vereint wird, gegebenenfalls unter Einsatz eines Lösemittels, wobei die Aldehydgruppen gegenüber den primären und sekundären Aminogruppen stöchiometrisch oder im stöchiometrischen Überschuss vorhanden sind, und
- das bei der Reaktion entstehende Kondensationswasser und gegebenenfalls eingesetztes Lösemittel während oder nach der Vereinigung mit einer geeigneten Methode aus der Reaktionsmischung entfernt werden, gegebenenfalls unter deren Erwärmen und/oder Anlegen von Vakuum.

11. Blockiertes Amin erhalten aus der Verwendung des Aldehydgemisches gemäss einem der Ansprüche 1 bis 10.

12. Blockiertes Amin gemäss Anspruch 11, **dadurch gekennzeichnet, dass** es funktionelle Gruppen der Formeln (II) und/oder (III) aufweist, 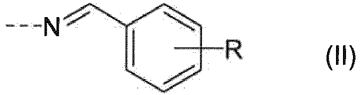 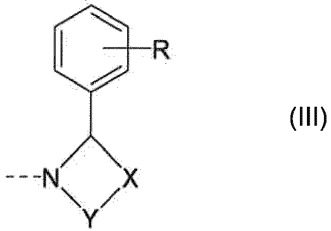 wobei
X für O oder S oder NR⁰ steht, wobei R° für einen einwertigen organischen Rest mit 1 bis 18 C-Atomen steht,
Y für einen 1,2-Ethylen- oder 1,3-Propylen-Rest, welcher gegebenenfalls substituiert ist, steht.

13. Isocyanatgruppen-haltige Zusammensetzung enthaltend mindestens ein blockiertes Amin gemäss einem der Ansprüche 11 bis 12.

14. Isocyanatgruppen-haltige Zusammensetzung gemäss Anspruch 13, **dadurch gekennzeichnet, dass** sie
- mindestens ein Polyisocyanat und/oder mindestens ein Isocyanatgruppen-haltiges Polyurethanpolymer, und
- mindestens ein Polyaldimin der Formel (IV), 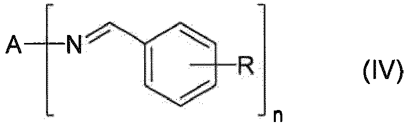
wobei
n für 2 oder 3 steht,
A für einen n-wertigen organischen Rest mit einem Molekulargewicht im Bereich von 28 bis 10'000 steht,
enthält.

## Claims

1. Use of an aldehyde mixture comprising
- 70% to 92% by weight of aldehydes of the Formula (I), in which R is an alkyl radical having 6 to 20 carbon atoms, and 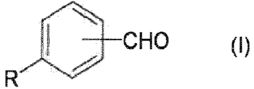
- 8% to 30% by weight of alkylbenzene compounds not corresponding to the Formula (I),
as blocking agent for amines, **characterized in that** the alkylbenzene compounds not corresponding to the Formula (I) have a higher boiling point than the aldehydes of the Formula (I).

2. Use according to Claim 1, **characterized in that** the alkyl radicals R are predominantly branched.

3. Use according to either of Claims 1 or 2, **characterized in that** the formyl group is in the para position.

4. Use according to one of Claims 1 to 3, **characterized in that** the aldehydes of the Formula (I) are selected from 4-decylbenzaldehydes, 4-undecylbenzaldehydes, 4-dodecylbenzaldehydes, 4-tridecylbenzaldehydes and 4-tetradecylbenzaldehydes, the alkyl radicals of which are predominantly branched.

5. Use according to one of Claims 1 to 4, **characterized in that** the alkylbenzene compounds not corresponding to the Formula (I) comprise one or more compounds of the formulae 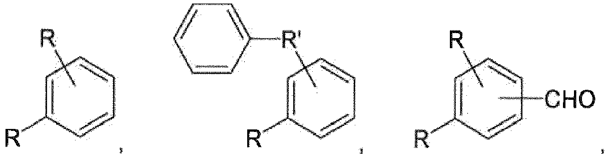 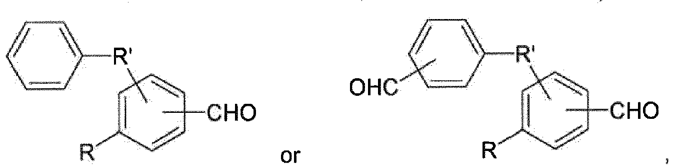 in which R' is an alkylene radical having 6 to 20 carbon atoms.

6. Use according to one of Claims 1 to 5, **characterized in that** the aldehyde mixture is a reaction product of a formylation of at least one alkybenzene of the formula

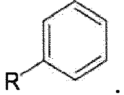

7. Use according to Claim 6, **characterized in that** the formylation was carried out with carbon monoxide in the presence of hydrofluoric acid and boron trifluoride.

8. Use according to one of Claims 1 to 7, **characterized in that** the amine to be blocked has at least one primary or secondary amino group and additionally at least one reactive group selected from primary amino group, secondary amino group, hydroxyl group and silane group.

9. Use according to one of Claims 1 to 8, **characterized in that** the amine to be blocked is selected from the group consisting of primary aliphatic diamines, primary aromatic diamines, primary aliphatic triamines, aliphatic diamines having a primary and a secondary amino group, aliphatic polyamines having two primary and a secondary amino group, aminoalcohols, dialkanolamines, aminosilanes and alkanolaminosilanes.

10. Use according to one of Claims 1 to 9, **characterized in that** the aldehyde mixture is reacted with the amine to be blocked so that
- the aldehyde mixture is combined with the amine to give a reaction mixture, optionally with addition of a solvent, the aldehyde groups being present, with regard to the primary and secondary amino groups, stoichiometrically or in stoichiometric excess, and
- the condensation water produced in the reaction and optionally solvent used during or after the combining are removed from the reaction mixture using a suitable method, optionally with heating of it and/or application of vacuum.

11. Blocked amine obtained from the use of the aldehyde mixture according to one of Claims 1 to 10.

12. Blocked amine according to Claim 11, **characterized in that** it has functional groups of the Formulae (II) and/or (III), 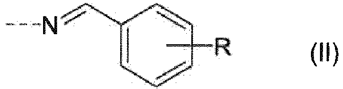 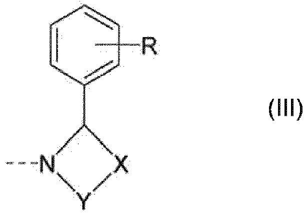 in which
X is O or S or NR⁰, in which R° is a monovalent organic radical having 1 to 18 carbon atoms,
Y is a 1,2-ethylene or 1,3-propylene radical, which is optionally substituted.

13. Composition containing isocyanate groups comprising at least one blocked amine according to one of Claims 11 to 12.

14. Composition containing isocyanate groups according to Claim 13, **characterized in that** it comprises
- at least one polyisocyanate and/or at least one polyurethane polymer containing isocyanate groups, and
- at least one polyaldimine of the Formula (IV), 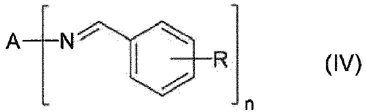
in which
n is 2 or 3,
A is an n-valent organic radical having a molecular weight in the range from 28 to 10 000.

## Revendications

1. Utilisation d'un mélange d'aldéhydes contenant
- 70 à 92% en poids d'aldéhydes de formule (I), R représentant un radical alkyle comportant 6 à 20 atomes de carbone et 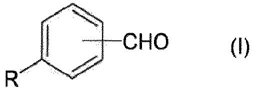
- 8 à 30% en poids de composés d'alkylbenzène ne correspondant pas à la formule (I)
comme agent de blocage pour des amines, **caractérisée en ce que** les composés d'alkylbenzène ne correspondant pas à la formule (I) présentent un point d'ébullition supérieur à ceux des aldéhydes de formule (I).

2. Utilisation selon la revendication 1, **caractérisée en ce que** les radicaux alkyle R sont à ramifications multiples.

3. Utilisation selon l'une quelconque des revendications 1 ou 2, **caractérisée en ce que** le groupe formyle se situe en position para.

4. Utilisation selon l'une quelconque des revendications 1 à 3, **caractérisée en ce que** les aldéhydes de formule (I) sont choisis parmi les 4-décylbenzaldéhydes, les 4-undécylbenzaldéhydes, les 4-dodécylbenzaldéhydes, les 4-tridécylbenzaldéhydes et les 4-tétradécylbenzaldéhydes, dont les radicaux alkyle sont à ramifications multiples.

5. Utilisation selon l'une quelconque des revendications 1 à 4, **caractérisée en ce que** les composés d'alkylbenzène ne correspondant pas à la formule (I) comprennent un ou plusieurs composés des formules 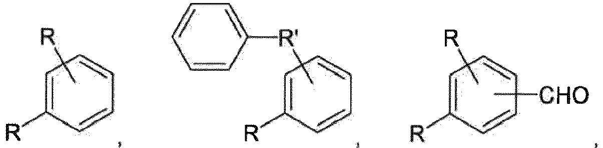 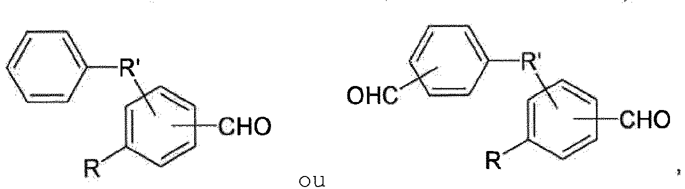 où R' représente un radical alkylène comportant 6 à 20 atomes de carbone.

6. Utilisation selon l'une quelconque des revendications 1 à 5, **caractérisée en ce que** le mélange d'aldéhydes représente un produit de transformation d'une formylation d'au moins un alkylbenzène de formule

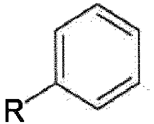

7. Utilisation selon la revendication 6, **caractérisée en ce que** la formylation a été réalisée avec du monoxyde de carbone en présence d'acide fluorhydrique et de trifluorure de bore.

8. Utilisation selon l'une quelconque des revendications 1 à 7, **caractérisée en ce que** l'amine à bloquer présente au moins un groupe amino primaire ou secondaire et en plus au moins un groupe réactif choisi parmi un groupe amino primaire, un groupe amino secondaire, un groupe hydroxyle et un groupe silane.

9. Utilisation selon l'une quelconque des revendications 1 à 8, **caractérisée en ce que** l'amine à bloquer est choisie dans le groupe constitué par les diamines aliphatiques primaires, les diamines aromatiques primaires, les triamines aliphatiques primaires, les diamines aliphatiques comportant un groupe amino primaire et un groupe amino secondaire, les polyamines aliphatiques comportant deux groupes amino primaire et un groupe amino secondaire, les aminoalcools, les dialcanolamines, les aminosilanes et les alcanolaminosilanes.

10. Utilisation selon l'une quelconque des revendications 1 à 9, **caractérisée en ce que** le mélange d'aldéhydes est transformé avec l'amine à bloquer de manière telle que
- le mélange d'aldéhydes est réuni avec l'amine en un mélange réactionnel, le cas échéant avec utilisation d'un solvant, les groupes aldéhyde étant présents en quantité stoechiométrique ou en excès stoechiométrique par rapport aux groupes amino primaire et secondaire et
- l'eau de condensation formée lors de la réaction et le solvant le cas échéant utilisé étant éliminés du mélange réactionnel pendant ou après la réunion à l'aide d'une méthode appropriée, le cas échéant par leur chauffage et/ou l'application d'un vide.

11. Amine bloquée obtenue à partir de l'utilisation du mélange d'aldéhydes selon l'une quelconque des revendications 1 à 10.

12. Amine bloquée selon la revendication 11, **caractérisée en ce qu'**elle présente des groupes fonctionnels des formules (II) et/ou (III), 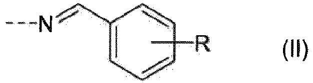 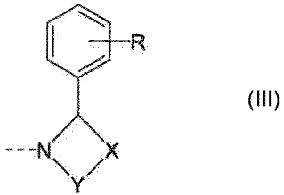 dans lesquelles
X représente O ou S ou NR⁰, R° représentant un radical organique monovalent, comportant 1 à 18 atomes de carbone,
Y représente un radical 1,2-éthylène ou 1,3-propylène, qui est le cas échéant substitué.

13. Composition contenant des groupes isocyanate contenant au moins une amine bloquée selon l'une quelconque des revendications 11 à 12.

14. Composition contenant des groupes isocyanate selon la revendication 13, **caractérisée en ce qu'**elle contient
- au moins un polyisocyanate et/ou au moins un polymère de polyuréthane contenant des groupes isocyanate et
- au moins une polyaldimine de formule (IV) 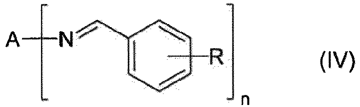
dans laquelle
n représente 1, 2 ou 3,
A représente un radical organique n-valent présentant un poids moléculaire dans la plage de 28 à 10.000.
